# EUROPEAN PATENT APPLICATION

(11) **EP 3 248 984 A2**
(43) Date of publication of application: **29.11.2017**
(21) Application number: 17165390.0
(22) Date of filing: 07.04.2017
(51) Int. Cl.: C07K 1/18

(54) **METHOD FOR PURIFYING PROTEIN, FUSION PROTEIN CONTAINING PEPTIDE TAG, AND PRODUCTION METHOD THEREOF**

(30) Priority: 07.04.2016 JP 2016077127
(71) Applicant: SYSMEX CORPORATION, Kobe-shi Hyogo 651-0073 (JP)
(72) Inventor: Takahashi, Ryo, Kobe-shi, Hyogo, 651-0073 (JP)
(74) Representative: De Clercq & Partners

(57) **Abstract**

Disclosed is a method for purifying a protein, comprising steps of: preparing a sample containing a fusion protein containing an amino acid sequence of a peptide tag and an amino acid sequence of a target protein; and separating contaminant proteins contained with the fusion protein in the sample and the fusion protein in the sample, wherein the peptide tag contains 12 or more acidic amino acid residues.

SEQUENCE LISTING

<110>  SYSMEX CORPORATION

<120>  Method of purifying protein,fusion protein comprising peptide tag,
and production method thereof

<130>  SYSM-117-EP

<140>  JP2016-077127
<141>  2016-04-07

<160>  67

<170>  PatentIn version 3.5

<210>  1
<211>  12
<212>  PRT
<213>  Artificial Sequence

<220>
<223>  DE12

<400>  1

Glu Glu Glu Glu Glu Glu Asp Asp Asp Asp Asp Asp
1               5                   10


<210>  2
<211>  18
<212>  PRT
<213>  Artificial Sequence

<220>
<223>  DE18

<400>  2

Glu Glu Glu Glu Glu Glu Glu Glu Glu Asp Asp Asp Asp Asp Asp Asp
1               5                   10                  15


Asp Asp



<210>  3
<211>  24
<212>  PRT
<213>  Artificial Sequence

<220>
<223>  DE24

<400>  3

Glu Glu Glu Glu Glu Glu Glu Glu Glu Glu Glu Glu Asp Asp Asp Asp
1               5                   10                  15


Asp Asp Asp Asp Asp Asp Asp Asp

            20

<210>  4
<211>  30
<212>  PRT
<213>  Artificial Sequence

<220>
<223>  DE30

<400>  4

Glu Glu Glu Glu Glu Glu Glu Glu Glu Glu Glu Glu Glu Glu Glu Asp
1               5                   10                  15

Asp Asp Asp Asp Asp Asp Asp Asp Asp Asp Asp Asp Asp Asp
            20                  25                  30

<210>  5
<211>  36
<212>  PRT
<213>  Artificial Sequence

<220>
<223>  DE36

<400>  5

Glu Glu Glu Glu Glu Glu Glu Glu Glu Glu Glu Glu Glu Glu Glu Glu
1               5                   10                  15

Glu Glu Asp Asp Asp Asp Asp Asp Asp Asp Asp Asp Asp Asp Asp Asp
            20                  25                  30

Asp Asp Asp Asp
        35

<210>  6
<211>  88
<212>  PRT
<213>  Artificial Sequence

<220>
<223>  DED

<400>  6

Asn Val Glu Gly Lys Thr Gly Asn Ala Thr Asp Glu Glu Glu Glu Glu
1               5                   10                  15

Glu Glu Glu Glu Glu Glu Glu Asp Asp Asp Asp Asp Asp Asp Asp Asp
            20                  25                  30

Asp Asp Asp Glu Asp Ser Gly Ala Glu Ile Gln Asp Asp Asp Glu Glu
        35                  40                  45

Gly Phe Asp Asp Glu Glu Glu Phe Asp Asp Asp Asp Asp Asp Glu His
    50                  55                  60

Asp Asp Asp Asp Leu Glu Asn Glu Glu Asn Glu Leu Glu Glu Leu Glu
65                  70                  75                  80

Glu Arg Val Glu Ala Arg Lys Lys
                85

<210>  7
<211>  44
<212>  PRT
<213>  Artificial Sequence

<220>
<223>  DES

<400>  7

Asp Leu Ser Asn Val Glu Gly Lys Thr Gly Asn Ala Thr Asp Glu Glu
1               5                   10                  15

Glu Glu Glu Glu Glu Glu Glu Glu Glu Glu Asp Asp Asp Asp Asp Asp
            20                  25                  30

Asp Asp Asp Asp Asp Asp Glu Asp Ser Gly Ala Glu
        35                  40

<210>  8
<211>  24
<212>  PRT
<213>  Artificial Sequence

<220>
<223>  EO24

<400>  8

Glu Glu Glu Glu Glu Glu Glu Glu Glu Glu Glu Glu Glu Glu Glu Glu
1               5                   10                  15

Glu Glu Glu Glu Glu Glu Glu Glu
            20

<210>  9
<211>  274
<212>  PRT
<213>  Artificial Sequence

<220>
<223>  NQ01

<400>  9

<210>  10
<211>  546
<212>  PRT
<213>  Artificial Sequence

<220>
<223>  Luciferase

<400>  10

Glu Asp Ala Lys Asn Ile Lys Lys Gly Pro Ala Pro Phe Tyr Pro Leu
1               5                   10                  15

Glu Asp Gly Thr Ala Gly Glu Gln Leu His Lys Ala Met Lys Arg Tyr
            20                  25                  30

Ala Leu Val Pro Gly Thr Ile Ala Phe Thr Asp Ala His Ile Glu Val
        35                  40                  45

Asn Ile Thr Tyr Ala Glu Tyr Phe Glu Met Ser Val Arg Leu Ala Glu
    50                  55                  60

Ala Met Lys Arg Tyr Gly Leu Asn Thr Asn His Arg Ile Val Val Cys
65                  70                  75                  80

Ser Glu Asn Ser Leu Gln Phe Phe Met Pro Val Leu Gly Ala Leu Phe
                85                  90                  95

Ile Gly Val Ala Val Ala Pro Ala Asn Asp Ile Tyr Asn Glu Arg Glu
            100                 105                 110

Leu Leu Asn Ser Met Asn Ile Ser Gln Pro Thr Val Val Phe Val Ser
        115                 120                 125

Lys Lys Gly Leu Gln Lys Ile Leu Asn Val Gln Lys Lys Leu Pro Ile
    130                 135                 140

Ile Gln Lys Ile Ile Ile Met Asp Ser Lys Thr Asp Tyr Gln Gly Phe
145                 150                 155                 160

Gln Ser Met Tyr Thr Phe Val Thr Ser His Leu Pro Pro Gly Phe Asn
                165                 170                 175

Glu Tyr Asp Phe Val Pro Glu Ser Phe Asp Arg Asp Lys Thr Ile Ala
            180                 185                 190

Leu Ile Met Asn Ser Ser Gly Ser Thr Gly Leu Pro Lys Gly Val Ala
        195                 200                 205

Leu Pro His Arg Thr Ala Cys Val Arg Phe Ser His Ala Arg Asp Pro
    210                 215                 220

Ile Phe Gly Asn Gln Ile Ile Pro Asp Thr Ala Ile Leu Ser Val Val
225                 230                 235                 240

Pro Phe His His Gly Phe Gly Met Phe Thr Thr Leu Gly Tyr Leu Ile
                245                 250                 255

Cys Gly Phe Arg Val Val Leu Met Tyr Arg Phe Glu Glu Glu Leu Phe
            260                 265                 270

Leu Arg Ser Leu Gln Asp Tyr Lys Ile Gln Ser Ala Leu Leu Val Pro
        275                 280                 285

Thr Leu Phe Ser Phe Phe Ala Lys Ser Thr Leu Ile Asp Lys Tyr Asp
    290                 295                 300

Leu Ser Asn Leu His Glu Ile Ala Ser Gly Gly Ala Pro Leu Ser Lys
305                 310                 315                 320

Glu Val Gly Glu Ala Val Ala Lys Arg Phe His Leu Pro Gly Ile Arg
                325                 330                 335

Gln Gly Tyr Gly Leu Thr Glu Thr Thr Ser Ala Ile Leu Ile Thr Pro
            340                 345                 350

Glu Gly Asp Asp Lys Pro Gly Ala Val Gly Lys Val Val Pro Phe Phe
        355                 360                 365

Glu Ala Lys Val Val Asp Leu Asp Thr Gly Lys Thr Leu Gly Val Asn
    370                 375                 380

Gln Arg Gly Glu Leu Cys Val Arg Gly Pro Met Ile Met Ser Gly Tyr
385                 390                 395                 400

Val Asn Asn Pro Glu Ala Thr Asn Ala Leu Ile Asp Lys Asp Gly Trp
                405                 410                 415

Leu His Ser Gly Asp Ile Ala Tyr Trp Asp Glu Asp Glu His Phe Phe
            420                 425                 430

Ile Val Asp Arg Leu Lys Ser Leu Ile Lys Tyr Lys Gly Tyr Gln Val
        435                 440                 445

Ala Pro Ala Glu Leu Glu Ser Ile Leu Leu Gln His Pro Asn Ile Phe
    450                 455                 460

Asp Ala Gly Val Ala Gly Leu Pro Asp Asp Asp Ala Gly Glu Leu Pro
465                 470                 475                 480

Ala Ala Val Val Val Leu Glu His Gly Lys Thr Met Thr Glu Lys Glu
                485                 490                 495

Ile Val Asp Tyr Val Ala Ser Gln Val Thr Thr Ala Lys Lys Leu Arg
            500                 505                 510

Gly Gly Val Val Phe Val Asp Glu Val Pro Lys Gly Leu Thr Gly Lys
        515                 520                 525

Leu Asp Ala Arg Lys Ile Arg Glu Ile Leu Ile Lys Ala Lys Lys Gly
    530                 535                 540

Gly Lys
545

<210>  11
<211>  202
<212>  PRT
<213>  Artificial Sequence

<220>  HRV3C

<400>  11

Asp Leu Val Pro Arg Gly Ser Pro Glu Phe Pro Gly Arg Leu Glu Arg
1               5                   10                  15

Pro His Arg Asp Gly Pro Asn Thr Glu Phe Ala Leu Ser Leu Leu Arg
            20                  25                  30

Lys Asn Ile Met Thr Ile Thr Thr Ser Lys Gly Glu Phe Thr Gly Leu
        35                  40                  45

Gly Ile His Asp Arg Val Cys Val Ile Pro Thr His Ala Gln Pro Gly
    50                  55                  60

Asp Asp Val Leu Val Asn Gly Gln Lys Ile Arg Val Lys Asp Lys Tyr
65                  70                  75                  80

Lys Leu Val Asp Pro Glu Asn Ile Asn Leu Glu Leu Thr Val Leu Thr
                85                  90                  95

Leu Asp Arg Asn Glu Lys Phe Arg Asp Ile Arg Gly Phe Ile Ser Glu
            100                 105                 110

Asp Leu Glu Gly Val Asp Ala Thr Leu Val Val His Ser Asn Asn Phe
        115                 120                 125

Thr Asn Thr Ile Leu Glu Val Gly Pro Val Thr Met Ala Gly Leu Ile
    130                 135                 140

Asn Leu Ser Ser Thr Pro Thr Asn Arg Met Ile Arg Tyr Asp Tyr Ala
145                 150                 155                 160

Thr Lys Thr Gly Gln Cys Gly Gly Val Leu Cys Ala Thr Gly Lys Ile
                165                 170                 175

Phe Gly Ile His Val Gly Gly Asn Gly Arg Gln Gly Phe Ser Ala Gln
            180                 185                 190

Leu Lys Lys Gln Tyr Phe Val Glu Lys Gln
        195                 200

<210>  12
<211>  298
<212>  PRT
<213>  Artificial Sequence

<220>
<223>  DE12-HRV3Csite-NQO1

<400>  12

Met Glu Glu Glu Glu Glu Glu Asp Asp Asp Asp Asp Asp Lys Gly Thr
1               5                   10                  15

Leu Glu Val Leu Phe Gln Gly Pro Met Val Gly Arg Arg Ala Leu Ile
            20                  25                  30

Val Leu Ala His Ser Glu Arg Thr Ser Phe Asn Tyr Ala Met Lys Glu
        35                  40                  45

Ala Ala Ala Ala Ala Leu Lys Lys Lys Gly Trp Glu Val Val Glu Ser
    50                  55                  60

Asp Leu Tyr Ala Met Asn Phe Asn Pro Ile Ile Ser Arg Lys Asp Ile
65                  70                  75                  80

Thr Gly Lys Leu Lys Asp Pro Ala Asn Phe Gln Tyr Pro Ala Glu Ser
                85                  90                  95

Val Leu Ala Tyr Lys Glu Gly His Leu Ser Pro Asp Ile Val Ala Glu
            100                 105                 110

Gln Lys Lys Leu Glu Ala Ala Asp Leu Val Ile Phe Gln Phe Pro Leu
        115                 120                 125

Gln Trp Phe Gly Val Pro Ala Ile Leu Lys Gly Trp Phe Glu Arg Val
    130                 135                 140

Phe Ile Gly Glu Phe Ala Tyr Thr Tyr Ala Ala Met Tyr Asp Lys Gly
145                 150                 155                 160

Pro Phe Arg Ser Lys Lys Ala Val Leu Ser Ile Thr Thr Gly Gly Ser
                165                 170                 175

Gly Ser Met Tyr Ser Leu Gln Gly Ile His Gly Asp Met Asn Val Ile
            180                 185                 190

Leu Trp Pro Ile Gln Ser Gly Ile Leu His Phe Cys Gly Phe Gln Val
        195                 200                 205

Leu Glu Pro Gln Leu Thr Tyr Ser Ile Gly His Thr Pro Ala Asp Ala
    210                 215                 220

Arg Ile Gln Ile Leu Glu Gly Trp Lys Lys Arg Leu Glu Asn Ile Trp
225                 230                 235                 240

Asp Glu Thr Pro Leu Tyr Phe Ala Pro Ser Ser Leu Phe Asp Leu Asn
                245                 250                 255

Phe Gln Ala Gly Phe Leu Met Lys Lys Glu Val Gln Asp Glu Glu Lys
            260                 265                 270

Asn Lys Lys Phe Gly Leu Ser Val Gly His His Leu Gly Lys Ser Ile
        275                 280                 285

Pro Thr Asp Asn Gln Ile Lys Ala Arg Lys
    290                 295

<210>  13
<211>  304
<212>  PRT
<213>  Artificial Sequence

<220>
<223>  DE18-HRV3Csite-NQO1

<400> 13
   Met Glu Glu Glu Glu Glu Glu Glu Glu Glu Asp Asp Asp Asp Asp Asp
   1               5                   10                  15
   Asp Asp Asp Lys Gly Thr Leu Glu Val Leu Phe Gln Gly Pro Met Val
               20                  25                  30
   Gly Arg Arg Ala Leu Ile Val Leu Ala His Ser Glu Arg Thr Ser Phe
           35                  40                  45
   Asn Tyr Ala Met Lys Glu Ala Ala Ala Ala Ala Leu Lys Lys Lys Gly
       50                  55                  60
   Trp Glu Val Val Glu Ser Asp Leu Tyr Ala Met Asn Phe Asn Pro Ile
   65                  70                  75                  80
   Ile Ser Arg Lys Asp Ile Thr Gly Lys Leu Lys Asp Pro Ala Asn Phe
                   85                  90                  95
   Gln Tyr Pro Ala Glu Ser Val Leu Ala Tyr Lys Glu Gly His Leu Ser
               100                 105                 110
   Pro Asp Ile Val Ala Glu Gln Lys Lys Leu Glu Ala Ala Asp Leu Val
           115                 120                 125
   Ile Phe Gln Phe Pro Leu Gln Trp Phe Gly Val Pro Ala Ile Leu Lys
       130                 135                 140
   Gly Trp Phe Glu Arg Val Phe Ile Gly Glu Phe Ala Tyr Thr Tyr Ala
   145                 150                 155                 160
   Ala Met Tyr Asp Lys Gly Pro Phe Arg Ser Lys Lys Ala Val Leu Ser
                   165                 170                 175
   Ile Thr Thr Gly Gly Ser Gly Ser Met Tyr Ser Leu Gln Gly Ile His
               180                 185                 190
   Gly Asp Met Asn Val Ile Leu Trp Pro Ile Gln Ser Gly Ile Leu His
           195                 200                 205
   Phe Cys Gly Phe Gln Val Leu Glu Pro Gln Leu Thr Tyr Ser Ile Gly
       210                 215                 220
   His Thr Pro Ala Asp Ala Arg Ile Gln Ile Leu Glu Gly Trp Lys Lys
   225                 230                 235                 240
   Arg Leu Glu Asn Ile Trp Asp Glu Thr Pro Leu Tyr Phe Ala Pro Ser
   245 250 255
   Ser Leu Phe Asp Leu Asn Phe Gln Ala Gly Phe Leu Met Lys Lys Glu
   260 265 270
   Val Gln Asp Glu Glu Lys Asn Lys Lys Phe Gly Leu Ser Val Gly His
   275 280 285
   His Leu Gly Lys Ser Ile Pro Thr Asp Asn Gln Ile Lys Ala Arg Lys
   290 295 300

<210>  14
<211>  310
<212>  PRT
<213>  Artificial Sequence

<220>
<223>  DE24-HRV3Csite-NQO1

<400>  14

Met Glu Glu Glu Glu Glu Glu Glu Glu Glu Glu Glu Glu Asp Asp Asp
1               5                   10                  15

Asp Asp Asp Asp Asp Asp Asp Asp Asp Lys Gly Thr Leu Glu Val Leu
            20                  25                  30

Phe Gln Gly Pro Met Val Gly Arg Arg Ala Leu Ile Val Leu Ala His
        35                  40                  45

Ser Glu Arg Thr Ser Phe Asn Tyr Ala Met Lys Glu Ala Ala Ala Ala
    50                  55                  60

Ala Leu Lys Lys Lys Gly Trp Glu Val Val Glu Ser Asp Leu Tyr Ala
65                  70                  75                  80

Met Asn Phe Asn Pro Ile Ile Ser Arg Lys Asp Ile Thr Gly Lys Leu
                85                  90                  95

Lys Asp Pro Ala Asn Phe Gln Tyr Pro Ala Glu Ser Val Leu Ala Tyr
            100                 105                 110

Lys Glu Gly His Leu Ser Pro Asp Ile Val Ala Glu Gln Lys Lys Leu
        115                 120                 125

Glu Ala Ala Asp Leu Val Ile Phe Gln Phe Pro Leu Gln Trp Phe Gly
    130                 135                 140

Val Pro Ala Ile Leu Lys Gly Trp Phe Glu Arg Val Phe Ile Gly Glu

145                 150                 155                 160

Phe Ala Tyr Thr Tyr Ala Ala Met Tyr Asp Lys Gly Pro Phe Arg Ser
                165                 170                 175

Lys Lys Ala Val Leu Ser Ile Thr Thr Gly Gly Ser Gly Ser Met Tyr
            180                 185                 190

Ser Leu Gln Gly Ile His Gly Asp Met Asn Val Ile Leu Trp Pro Ile
        195                 200                 205

Gln Ser Gly Ile Leu His Phe Cys Gly Phe Gln Val Leu Glu Pro Gln
    210                 215                 220

Leu Thr Tyr Ser Ile Gly His Thr Pro Ala Asp Ala Arg Ile Gln Ile
225                 230                 235                 240

Leu Glu Gly Trp Lys Lys Arg Leu Glu Asn Ile Trp Asp Glu Thr Pro
                245                 250                 255

Leu Tyr Phe Ala Pro Ser Ser Leu Phe Asp Leu Asn Phe Gln Ala Gly
            260                 265                 270

Phe Leu Met Lys Lys Glu Val Gln Asp Glu Glu Lys Asn Lys Lys Phe
        275                 280                 285

Gly Leu Ser Val Gly His His Leu Gly Lys Ser Ile Pro Thr Asp Asn
    290                 295                 300

Gln Ile Lys Ala Arg Lys
305                 310

<210>  15
<211>  316
<212>  PRT
<213>  Artificial Sequence

<220>
<223>  DE30-HRV3Csite-NQO1

<400>  15

Met Glu Glu Glu Glu Glu Glu Glu Glu Glu Glu Glu Glu Glu Glu Glu
1               5                   10                  15

Asp Asp Asp Asp Asp Asp Asp Asp Asp Asp Asp Asp Asp Asp Asp Lys
            20                  25                  30

Gly Thr Leu Glu Val Leu Phe Gln Gly Pro Met Val Gly Arg Arg Ala
        35                  40                  45

Leu Ile Val Leu Ala His Ser Glu Arg Thr Ser Phe Asn Tyr Ala Met
    50                  55                  60

Lys Glu Ala Ala Ala Ala Ala Leu Lys Lys Lys Gly Trp Glu Val Val
65                  70                  75                  80

Glu Ser Asp Leu Tyr Ala Met Asn Phe Asn Pro Ile Ile Ser Arg Lys
                85                  90                  95

Asp Ile Thr Gly Lys Leu Lys Asp Pro Ala Asn Phe Gln Tyr Pro Ala
            100                 105                 110

Glu Ser Val Leu Ala Tyr Lys Glu Gly His Leu Ser Pro Asp Ile Val
        115                 120                 125

Ala Glu Gln Lys Lys Leu Glu Ala Ala Asp Leu Val Ile Phe Gln Phe
    130                 135                 140

Pro Leu Gln Trp Phe Gly Val Pro Ala Ile Leu Lys Gly Trp Phe Glu
145                 150                 155                 160

Arg Val Phe Ile Gly Glu Phe Ala Tyr Thr Tyr Ala Ala Met Tyr Asp
                165                 170                 175

Lys Gly Pro Phe Arg Ser Lys Lys Ala Val Leu Ser Ile Thr Thr Gly
            180                 185                 190

Gly Ser Gly Ser Met Tyr Ser Leu Gln Gly Ile His Gly Asp Met Asn
        195                 200                 205

Val Ile Leu Trp Pro Ile Gln Ser Gly Ile Leu His Phe Cys Gly Phe
    210                 215                 220

Gln Val Leu Glu Pro Gln Leu Thr Tyr Ser Ile Gly His Thr Pro Ala
225                 230                 235                 240

Asp Ala Arg Ile Gln Ile Leu Glu Gly Trp Lys Lys Arg Leu Glu Asn
                245                 250                 255

Ile Trp Asp Glu Thr Pro Leu Tyr Phe Ala Pro Ser Ser Leu Phe Asp
            260                 265                 270

Leu Asn Phe Gln Ala Gly Phe Leu Met Lys Lys Glu Val Gln Asp Glu
        275                 280                 285

Glu Lys Asn Lys Lys Phe Gly Leu Ser Val Gly His His Leu Gly Lys

290                 295                 300

Ser Ile Pro Thr Asp Asn Gln Ile Lys Ala Arg Lys
305                 310                 315

<210>  16
<211>  322
<212>  PRT
<213>  Artificial Sequence

<220>
<223>  DE36-HRV3Csite-NQO1

<400>  16

Met Glu Glu Glu Glu Glu Glu Glu Glu Glu Glu Glu Glu Glu Glu Glu
1               5                   10                  15

Glu Glu Glu Asp Asp Asp Asp Asp Asp Asp Asp Asp Asp Asp Asp Asp
            20                  25                  30

Asp Asp Asp Asp Asp Lys Gly Thr Leu Glu Val Leu Phe Gln Gly Pro
        35                  40                  45

Met Val Gly Arg Arg Ala Leu Ile Val Leu Ala His Ser Glu Arg Thr
    50                  55                  60

Ser Phe Asn Tyr Ala Met Lys Glu Ala Ala Ala Ala Ala Leu Lys Lys
65                  70                  75                  80

Lys Gly Trp Glu Val Val Glu Ser Asp Leu Tyr Ala Met Asn Phe Asn
                85                  90                  95

Pro Ile Ile Ser Arg Lys Asp Ile Thr Gly Lys Leu Lys Asp Pro Ala
            100                 105                 110

Asn Phe Gln Tyr Pro Ala Glu Ser Val Leu Ala Tyr Lys Glu Gly His
        115                 120                 125

Leu Ser Pro Asp Ile Val Ala Glu Gln Lys Lys Leu Glu Ala Ala Asp
    130                 135                 140

Leu Val Ile Phe Gln Phe Pro Leu Gln Trp Phe Gly Val Pro Ala Ile
145                 150                 155                 160

Leu Lys Gly Trp Phe Glu Arg Val Phe Ile Gly Glu Phe Ala Tyr Thr
                165                 170                 175

Tyr Ala Ala Met Tyr Asp Lys Gly Pro Phe Arg Ser Lys Lys Ala Val
            180                 185                 190

Leu Ser Ile Thr Thr Gly Gly Ser Gly Ser Met Tyr Ser Leu Gln Gly
        195                 200                 205

Ile His Gly Asp Met Asn Val Ile Leu Trp Pro Ile Gln Ser Gly Ile
    210                 215                 220

Leu His Phe Cys Gly Phe Gln Val Leu Glu Pro Gln Leu Thr Tyr Ser
225                 230                 235                 240

Ile Gly His Thr Pro Ala Asp Ala Arg Ile Gln Ile Leu Glu Gly Trp
                245                 250                 255

Lys Lys Arg Leu Glu Asn Ile Trp Asp Glu Thr Pro Leu Tyr Phe Ala
            260                 265                 270

Pro Ser Ser Leu Phe Asp Leu Asn Phe Gln Ala Gly Phe Leu Met Lys
        275                 280                 285

Lys Glu Val Gln Asp Glu Glu Lys Asn Lys Lys Phe Gly Leu Ser Val
    290                 295                 300

Gly His His Leu Gly Lys Ser Ile Pro Thr Asp Asn Gln Ile Lys Ala
305                 310                 315                 320

Arg Lys

<210>  17
<211>  388
<212>  PRT
<213>  Artificial Sequence

<220>
<223>  DED-HRV3Csite-NQO1

<400>  17

Met Asp Tyr Lys Asp Asp Asp Asp Lys Gly Met Ala Ser Asp Leu Ser
1               5                   10                  15

Asn Val Glu Gly Lys Thr Gly Asn Ala Thr Asp Glu Glu Glu Glu Glu
            20                  25                  30

Glu Glu Glu Glu Glu Glu Glu Asp Asp Asp Asp Asp Asp Asp Asp Asp
        35                  40                  45

Asp Asp Asp Glu Asp Ser Gly Ala Glu Ile Gln Asp Asp Asp Glu Glu
    50                  55                  60

Gly Phe Asp Asp Glu Glu Glu Phe Asp Asp Asp Asp Asp Asp Glu His
65                  70                  75                  80

Asp Asp Asp Asp Leu Glu Asn Glu Glu Asn Glu Leu Glu Glu Leu Glu
                85                  90                  95

Glu Arg Val Glu Ala Arg Lys Lys Ala Ser Leu Glu Val Leu Phe Gln
            100                 105                 110

Gly Pro Met Val Gly Arg Arg Ala Leu Ile Val Leu Ala His Ser Glu
        115                 120                 125

Arg Thr Ser Phe Asn Tyr Ala Met Lys Glu Ala Ala Ala Ala Ala Leu
    130                 135                 140

Lys Lys Lys Gly Trp Glu Val Val Glu Ser Asp Leu Tyr Ala Met Asn
145                 150                 155                 160

Phe Asn Pro Ile Ile Ser Arg Lys Asp Ile Thr Gly Lys Leu Lys Asp
                165                 170                 175

Pro Ala Asn Phe Gln Tyr Pro Ala Glu Ser Val Leu Ala Tyr Lys Glu
            180                 185                 190

Gly His Leu Ser Pro Asp Ile Val Ala Glu Gln Lys Lys Leu Glu Ala
        195                 200                 205

Ala Asp Leu Val Ile Phe Gln Phe Pro Leu Gln Trp Phe Gly Val Pro
    210                 215                 220

Ala Ile Leu Lys Gly Trp Phe Glu Arg Val Phe Ile Gly Glu Phe Ala
225                 230                 235                 240

Tyr Thr Tyr Ala Ala Met Tyr Asp Lys Gly Pro Phe Arg Ser Lys Lys
                245                 250                 255

Ala Val Leu Ser Ile Thr Thr Gly Gly Ser Gly Ser Met Tyr Ser Leu
            260                 265                 270

Gln Gly Ile His Gly Asp Met Asn Val Ile Leu Trp Pro Ile Gln Ser
        275                 280                 285

Gly Ile Leu His Phe Cys Gly Phe Gln Val Leu Glu Pro Gln Leu Thr
    290                 295                 300

Tyr Ser Ile Gly His Thr Pro Ala Asp Ala Arg Ile Gln Ile Leu Glu
305                 310                 315                 320

Gly Trp Lys Lys Arg Leu Glu Asn Ile Trp Asp Glu Thr Pro Leu Tyr
                325                 330                 335

Phe Ala Pro Ser Ser Leu Phe Asp Leu Asn Phe Gln Ala Gly Phe Leu
            340                 345                 350

Met Lys Lys Glu Val Gln Asp Glu Glu Lys Asn Lys Lys Phe Gly Leu
        355                 360                 365

Ser Val Gly His His Leu Gly Lys Ser Ile Pro Thr Asp Asn Gln Ile
    370                 375                 380

Lys Ala Arg Lys
385

<210>  18
<211>  341
<212>  PRT
<213>  Artificial Sequence

<220>
<223>  DES-HRV3Csite-NQO1

<400>  18

Met Asp Tyr Lys Asp Asp Asp Asp Lys Gly Met Ala Ser Asp Leu Ser
1               5                   10                  15

Asn Val Glu Gly Lys Thr Gly Asn Ala Thr Asp Glu Glu Glu Glu Glu
            20                  25                  30

Glu Glu Glu Glu Glu Glu Glu Asp Asp Asp Asp Asp Asp Asp Asp Asp
        35                  40                  45

Asp Asp Asp Glu Asp Ser Gly Ala Glu Ala Ser Leu Glu Val Leu Phe
    50                  55                  60

Gln Gly Pro Met Val Gly Arg Arg Ala Leu Ile Val Leu Ala His Ser
65                  70                  75                  80

Glu Arg Thr Ser Phe Asn Tyr Ala Met Lys Glu Ala Ala Ala Ala Ala
                85                  90                  95

Leu Lys Lys Lys Gly Trp Glu Val Val Glu Ser Asp Leu Tyr Ala Met
            100                 105                 110

Asn Phe Asn Pro Ile Ile Ser Arg Lys Asp Ile Thr Gly Lys Leu Lys
        115                 120                 125

Asp Pro Ala Asn Phe Gln Tyr Pro Ala Glu Ser Val Leu Ala Tyr Lys
    130                 135                 140

Glu Gly His Leu Ser Pro Asp Ile Val Ala Glu Gln Lys Lys Leu Glu
145                 150                 155                 160

Ala Ala Asp Leu Val Ile Phe Gln Phe Pro Leu Gln Trp Phe Gly Val
                165                 170                 175

Pro Ala Ile Leu Lys Gly Trp Phe Glu Arg Val Phe Ile Gly Glu Phe
            180                 185                 190

Ala Tyr Thr Tyr Ala Ala Met Tyr Asp Lys Gly Pro Phe Arg Ser Lys
        195                 200                 205

Lys Ala Val Leu Ser Ile Thr Thr Gly Gly Ser Gly Ser Met Tyr Ser
    210                 215                 220

Leu Gln Gly Ile His Gly Asp Met Asn Val Ile Leu Trp Pro Ile Gln
225                 230                 235                 240

Ser Gly Ile Leu His Phe Cys Gly Phe Gln Val Leu Glu Pro Gln Leu
                245                 250                 255

Thr Tyr Ser Ile Gly His Thr Pro Ala Asp Ala Arg Ile Gln Ile Leu
            260                 265                 270

Glu Gly Trp Lys Lys Arg Leu Glu Asn Ile Trp Asp Glu Thr Pro Leu
        275                 280                 285

Tyr Phe Ala Pro Ser Ser Leu Phe Asp Leu Asn Phe Gln Ala Gly Phe
    290                 295                 300

Leu Met Lys Lys Glu Val Gln Asp Glu Glu Lys Asn Lys Lys Phe Gly
305                 310                 315                 320

Leu Ser Val Gly His His Leu Gly Lys Ser Ile Pro Thr Asp Asn Gln
                325                 330                 335

Ile Lys Ala Arg Lys
            340

<210>  19
<211>  321
<212>  PRT
<213>  Artificial Sequence

<220>

<223>  EO24-HRV3Csite-NQO1

<400>  19

Met Asp Tyr Lys Asp Asp Asp Asp Lys Gly Met Ala Ser Glu Glu Glu
1               5                   10                  15

Glu Glu Glu Glu Glu Glu Glu Glu Glu Glu Glu Glu Glu Glu Glu Glu
            20                  25                  30

Glu Glu Glu Glu Glu Ala Ser Leu Glu Val Leu Phe Gln Gly Pro Met
        35                  40                  45

Val Gly Arg Arg Ala Leu Ile Val Leu Ala His Ser Glu Arg Thr Ser
    50                  55                  60

Phe Asn Tyr Ala Met Lys Glu Ala Ala Ala Ala Ala Leu Lys Lys Lys
65                  70                  75                  80

Gly Trp Glu Val Val Glu Ser Asp Leu Tyr Ala Met Asn Phe Asn Pro
                85                  90                  95

Ile Ile Ser Arg Lys Asp Ile Thr Gly Lys Leu Lys Asp Pro Ala Asn
            100                 105                 110

Phe Gln Tyr Pro Ala Glu Ser Val Leu Ala Tyr Lys Glu Gly His Leu
        115                 120                 125

Ser Pro Asp Ile Val Ala Glu Gln Lys Lys Leu Glu Ala Ala Asp Leu
    130                 135                 140

Val Ile Phe Gln Phe Pro Leu Gln Trp Phe Gly Val Pro Ala Ile Leu
145                 150                 155                 160

Lys Gly Trp Phe Glu Arg Val Phe Ile Gly Glu Phe Ala Tyr Thr Tyr
                165                 170                 175

Ala Ala Met Tyr Asp Lys Gly Pro Phe Arg Ser Lys Lys Ala Val Leu
            180                 185                 190

Ser Ile Thr Thr Gly Gly Ser Gly Ser Met Tyr Ser Leu Gln Gly Ile
        195                 200                 205

His Gly Asp Met Asn Val Ile Leu Trp Pro Ile Gln Ser Gly Ile Leu
    210                 215                 220

His Phe Cys Gly Phe Gln Val Leu Glu Pro Gln Leu Thr Tyr Ser Ile
225                 230                 235                 240

Gly His Thr Pro Ala Asp Ala Arg Ile Gln Ile Leu Glu Gly Trp Lys
                245                 250                 255

Lys Arg Leu Glu Asn Ile Trp Asp Glu Thr Pro Leu Tyr Phe Ala Pro
            260                 265                 270

Ser Ser Leu Phe Asp Leu Asn Phe Gln Ala Gly Phe Leu Met Lys Lys
        275                 280                 285

Glu Val Gln Asp Glu Glu Lys Asn Lys Lys Phe Gly Leu Ser Val Gly
    290                 295                 300

His His Leu Gly Lys Ser Ile Pro Thr Asp Asn Gln Ile Lys Ala Arg
305                 310                 315                 320

Lys

<210>  20
<211>  386
<212>  PRT
<213>  Artificial Sequence

<220>
<223>  NQO1-HRV3Csite-DED

<400>  20

Met Val Gly Arg Arg Ala Leu Ile Val Leu Ala His Ser Glu Arg Thr
1               5                   10                  15

Ser Phe Asn Tyr Ala Met Lys Glu Ala Ala Ala Ala Ala Leu Lys Lys
            20                  25                  30

Lys Gly Trp Glu Val Val Glu Ser Asp Leu Tyr Ala Met Asn Phe Asn
        35                  40                  45

Pro Ile Ile Ser Arg Lys Asp Ile Thr Gly Lys Leu Lys Asp Pro Ala
    50                  55                  60

Asn Phe Gln Tyr Pro Ala Glu Ser Val Leu Ala Tyr Lys Glu Gly His
65                  70                  75                  80

Leu Ser Pro Asp Ile Val Ala Glu Gln Lys Lys Leu Glu Ala Ala Asp
                85                  90                  95

Leu Val Ile Phe Gln Phe Pro Leu Gln Trp Phe Gly Val Pro Ala Ile
            100                 105                 110

Leu Lys Gly Trp Phe Glu Arg Val Phe Ile Gly Glu Phe Ala Tyr Thr
        115                 120                 125

Tyr Ala Ala Met Tyr Asp Lys Gly Pro Phe Arg Ser Lys Lys Ala Val
    130                 135                 140

Leu Ser Ile Thr Thr Gly Gly Ser Gly Ser Met Tyr Ser Leu Gln Gly
145                 150                 155                 160

Ile His Gly Asp Met Asn Val Ile Leu Trp Pro Ile Gln Ser Gly Ile
                165                 170                 175

Leu His Phe Cys Gly Phe Gln Val Leu Glu Pro Gln Leu Thr Tyr Ser
            180                 185                 190

Ile Gly His Thr Pro Ala Asp Ala Arg Ile Gln Ile Leu Glu Gly Trp
        195                 200                 205

Lys Lys Arg Leu Glu Asn Ile Trp Asp Glu Thr Pro Leu Tyr Phe Ala
    210                 215                 220

Pro Ser Ser Leu Phe Asp Leu Asn Phe Gln Ala Gly Phe Leu Met Lys
225                 230                 235                 240

Lys Glu Val Gln Asp Glu Glu Lys Asn Lys Lys Phe Gly Leu Ser Val
                245                 250                 255

Gly His His Leu Gly Lys Ser Ile Pro Thr Asp Asn Gln Ile Lys Ala
            260                 265                 270

Arg Lys Gly Leu Glu Val Leu Phe Gln Gly Pro Ala Ser Asp Leu Ser
        275                 280                 285

Asn Val Glu Gly Lys Thr Gly Asn Ala Thr Asp Glu Glu Glu Glu Glu
    290                 295                 300

Glu Glu Glu Glu Glu Glu Glu Asp Asp Asp Asp Asp Asp Asp Asp Asp
305                 310                 315                 320

Asp Asp Asp Glu Asp Ser Gly Ala Glu Ile Gln Asp Asp Asp Glu Glu
                325                 330                 335

Gly Phe Asp Asp Glu Glu Glu Phe Asp Asp Asp Asp Asp Asp Glu His
            340                 345                 350

Asp Asp Asp Asp Leu Glu Asn Glu Glu Asn Glu Leu Glu Glu Leu Glu
        355                 360                 365

Glu Arg Val Glu Ala Arg Lys Lys Ala Ser Asp Tyr Lys Asp Asp Asp
    370                 375                 380

Asp Lys
385

<210>  21
<211>  339
<212>  PRT
<213>  Artificial Sequence

<220>
<223>  NQO1-HRV3Csite-DES

<400>  21

Met Val Gly Arg Arg Ala Leu Ile Val Leu Ala His Ser Glu Arg Thr
1               5                   10                  15

Ser Phe Asn Tyr Ala Met Lys Glu Ala Ala Ala Ala Ala Leu Lys Lys
            20                  25                  30

Lys Gly Trp Glu Val Val Glu Ser Asp Leu Tyr Ala Met Asn Phe Asn
        35                  40                  45

Pro Ile Ile Ser Arg Lys Asp Ile Thr Gly Lys Leu Lys Asp Pro Ala
    50                  55                  60

Asn Phe Gln Tyr Pro Ala Glu Ser Val Leu Ala Tyr Lys Glu Gly His
65                  70                  75                  80

Leu Ser Pro Asp Ile Val Ala Glu Gln Lys Lys Leu Glu Ala Ala Asp
                85                  90                  95

Leu Val Ile Phe Gln Phe Pro Leu Gln Trp Phe Gly Val Pro Ala Ile
            100                 105                 110

Leu Lys Gly Trp Phe Glu Arg Val Phe Ile Gly Glu Phe Ala Tyr Thr
        115                 120                 125

Tyr Ala Ala Met Tyr Asp Lys Gly Pro Phe Arg Ser Lys Lys Ala Val
    130                 135                 140

Leu Ser Ile Thr Thr Gly Gly Ser Gly Ser Met Tyr Ser Leu Gln Gly
145                 150                 155                 160

Ile His Gly Asp Met Asn Val Ile Leu Trp Pro Ile Gln Ser Gly Ile
                165                 170                 175

Leu His Phe Cys Gly Phe Gln Val Leu Glu Pro Gln Leu Thr Tyr Ser
            180                 185                 190

Ile Gly His Thr Pro Ala Asp Ala Arg Ile Gln Ile Leu Glu Gly Trp
        195                 200                 205

Lys Lys Arg Leu Glu Asn Ile Trp Asp Glu Thr Pro Leu Tyr Phe Ala
    210                 215                 220

Pro Ser Ser Leu Phe Asp Leu Asn Phe Gln Ala Gly Phe Leu Met Lys
225                 230                 235                 240

Lys Glu Val Gln Asp Glu Glu Lys Asn Lys Lys Phe Gly Leu Ser Val
                245                 250                 255

Gly His His Leu Gly Lys Ser Ile Pro Thr Asp Asn Gln Ile Lys Ala
            260                 265                 270

Arg Lys Gly Leu Glu Val Leu Phe Gln Gly Pro Ala Ser Asp Leu Ser
        275                 280                 285

Asn Val Glu Gly Lys Thr Gly Asn Ala Thr Asp Glu Glu Glu Glu Glu
    290                 295                 300

Glu Glu Glu Glu Glu Glu Glu Asp Asp Asp Asp Asp Asp Asp Asp Asp
305                 310                 315                 320

Asp Asp Asp Glu Asp Ser Gly Ala Glu Ala Ser Asp Tyr Lys Asp Asp
                325                 330                 335

Asp Asp Lys

<210>  22
<211>  660
<212>  PRT
<213>  Artificial Sequence

<220>
<223>  DED-HRV3Csite-Luciferase

<400>  22

Met Asp Tyr Lys Asp Asp Asp Asp Lys Gly Met Ala Ser Asp Leu Ser
1               5                   10                  15

Asn Val Glu Gly Lys Thr Gly Asn Ala Thr Asp Glu Glu Glu Glu Glu
            20                  25                  30

Glu Glu Glu Glu Glu Glu Glu Asp Asp Asp Asp Asp Asp Asp Asp Asp

        35                  40                  45

Asp Asp Asp Glu Asp Ser Gly Ala Glu Ile Gln Asp Asp Asp Glu Glu
    50                  55                  60

Gly Phe Asp Asp Glu Glu Glu Phe Asp Asp Asp Asp Asp Asp Glu His
65                  70                  75                  80

Asp Asp Asp Asp Leu Glu Asn Glu Glu Asn Glu Leu Glu Glu Leu Glu
                85                  90                  95

Glu Arg Val Glu Ala Arg Lys Lys Ala Ser Leu Glu Val Leu Phe Gln
            100                 105                 110

Gly Pro Glu Asp Ala Lys Asn Ile Lys Lys Gly Pro Ala Pro Phe Tyr
        115                 120                 125

Pro Leu Glu Asp Gly Thr Ala Gly Glu Gln Leu His Lys Ala Met Lys
    130                 135                 140

Arg Tyr Ala Leu Val Pro Gly Thr Ile Ala Phe Thr Asp Ala His Ile
145                 150                 155                 160

Glu Val Asn Ile Thr Tyr Ala Glu Tyr Phe Glu Met Ser Val Arg Leu
                165                 170                 175

Ala Glu Ala Met Lys Arg Tyr Gly Leu Asn Thr Asn His Arg Ile Val
            180                 185                 190

Val Cys Ser Glu Asn Ser Leu Gln Phe Phe Met Pro Val Leu Gly Ala
        195                 200                 205

Leu Phe Ile Gly Val Ala Val Ala Pro Ala Asn Asp Ile Tyr Asn Glu
    210                 215                 220

Arg Glu Leu Leu Asn Ser Met Asn Ile Ser Gln Pro Thr Val Val Phe
225                 230                 235                 240

Val Ser Lys Lys Gly Leu Gln Lys Ile Leu Asn Val Gln Lys Lys Leu
                245                 250                 255

Pro Ile Ile Gln Lys Ile Ile Ile Met Asp Ser Lys Thr Asp Tyr Gln
            260                 265                 270

Gly Phe Gln Ser Met Tyr Thr Phe Val Thr Ser His Leu Pro Pro Gly
        275                 280                 285

Phe Asn Glu Tyr Asp Phe Val Pro Glu Ser Phe Asp Arg Asp Lys Thr
    290                 295                 300

Ile Ala Leu Ile Met Asn Ser Ser Gly Ser Thr Gly Leu Pro Lys Gly
305                 310                 315                 320

Val Ala Leu Pro His Arg Thr Ala Cys Val Arg Phe Ser His Ala Arg
                325                 330                 335

Asp Pro Ile Phe Gly Asn Gln Ile Ile Pro Asp Thr Ala Ile Leu Ser
            340                 345                 350

Val Val Pro Phe His His Gly Phe Gly Met Phe Thr Thr Leu Gly Tyr
        355                 360                 365

Leu Ile Cys Gly Phe Arg Val Val Leu Met Tyr Arg Phe Glu Glu Glu
    370                 375                 380

Leu Phe Leu Arg Ser Leu Gln Asp Tyr Lys Ile Gln Ser Ala Leu Leu
385                 390                 395                 400

Val Pro Thr Leu Phe Ser Phe Phe Ala Lys Ser Thr Leu Ile Asp Lys
                405                 410                 415

Tyr Asp Leu Ser Asn Leu His Glu Ile Ala Ser Gly Gly Ala Pro Leu
            420                 425                 430

Ser Lys Glu Val Gly Glu Ala Val Ala Lys Arg Phe His Leu Pro Gly
        435                 440                 445

Ile Arg Gln Gly Tyr Gly Leu Thr Glu Thr Thr Ser Ala Ile Leu Ile
    450                 455                 460

Thr Pro Glu Gly Asp Asp Lys Pro Gly Ala Val Gly Lys Val Val Pro
465                 470                 475                 480

Phe Phe Glu Ala Lys Val Val Asp Leu Asp Thr Gly Lys Thr Leu Gly
                485                 490                 495

Val Asn Gln Arg Gly Glu Leu Cys Val Arg Gly Pro Met Ile Met Ser
            500                 505                 510

Gly Tyr Val Asn Asn Pro Glu Ala Thr Asn Ala Leu Ile Asp Lys Asp
        515                 520                 525

Gly Trp Leu His Ser Gly Asp Ile Ala Tyr Trp Asp Glu Asp Glu His
    530                 535                 540

Phe Phe Ile Val Asp Arg Leu Lys Ser Leu Ile Lys Tyr Lys Gly Tyr
545                 550                 555                 560

Gln Val Ala Pro Ala Glu Leu Glu Ser Ile Leu Leu Gln His Pro Asn
                565                 570                 575

Ile Phe Asp Ala Gly Val Ala Gly Leu Pro Asp Asp Asp Ala Gly Glu
            580                 585                 590

Leu Pro Ala Ala Val Val Val Leu Glu His Gly Lys Thr Met Thr Glu
        595                 600                 605

Lys Glu Ile Val Asp Tyr Val Ala Ser Gln Val Thr Thr Ala Lys Lys
    610                 615                 620

Leu Arg Gly Gly Val Val Phe Val Asp Glu Val Pro Lys Gly Leu Thr
625                 630                 635                 640

Gly Lys Leu Asp Ala Arg Lys Ile Arg Glu Ile Leu Ile Lys Ala Lys
                645                 650                 655

Lys Gly Gly Lys
            660

<210>  23
<211>  613
<212>  PRT
<213>  Artificial Sequence

<220>
<223>  DES-HRV3Csite-Luciferase

<400>  23
   Met Asp Tyr Lys Asp Asp Asp Asp Lys Gly Met Ala Ser Asp Leu Ser
   1               5                   10                  15
   Asn Val Glu Gly Lys Thr Gly Asn Ala Thr Asp Glu Glu Glu Glu Glu
               20                  25                  30
   Glu Glu Glu Glu Glu Glu Glu Asp Asp Asp Asp Asp Asp Asp Asp Asp
           35                  40                  45
   Asp Asp Asp Glu Asp Ser Gly Ala Glu Ala Ser Leu Glu Val Leu Phe
       50                  55                  60
   Gln Gly Pro Glu Asp Ala Lys Asn Ile Lys Lys Gly Pro Ala Pro Phe
   65                  70                  75                  80
   Tyr Pro Leu Glu Asp Gly Thr Ala Gly Glu Gln Leu His Lys Ala Met
                   85                  90                  95
   Lys Arg Tyr Ala Leu Val Pro Gly Thr Ile Ala Phe Thr Asp Ala His
               100                 105                 110
   Ile Glu Val Asn Ile Thr Tyr Ala Glu Tyr Phe Glu Met Ser Val Arg
           115                 120                 125
   Leu Ala Glu Ala Met Lys Arg Tyr Gly Leu Asn Thr Asn His Arg Ile
       130                 135                 140
   Val Val Cys Ser Glu Asn Ser Leu Gln Phe Phe Met Pro Val Leu Gly
   145                 150                 155                 160
   Ala Leu Phe Ile Gly Val Ala Val Ala Pro Ala Asn Asp Ile Tyr Asn
                   165                 170                 175
   Glu Arg Glu Leu Leu Asn Ser Met Asn Ile Ser Gln Pro Thr Val Val
               180                 185                 190
   Phe Val Ser Lys Lys Gly Leu Gln Lys Ile Leu Asn Val Gln Lys Lys
           195                 200                 205
   Leu Pro Ile Ile Gln Lys Ile Ile Ile Met Asp Ser Lys Thr Asp Tyr
       210                 215                 220
   Gln Gly Phe Gln Ser Met Tyr Thr Phe Val Thr Ser His Leu Pro Pro
   225                 230                 235                 240
   Gly Phe Asn Glu Tyr Asp Phe Val Pro Glu Ser Phe Asp Arg Asp Lys
                   245                 250                 255
   Thr Ile Ala Leu Ile Met Asn Ser Ser Gly Ser Thr Gly Leu Pro Lys
               260                 265                 270
   Gly Val Ala Leu Pro His Arg Thr Ala Cys Val Arg Phe Ser His Ala
           275                 280                 285
   Arg Asp Pro Ile Phe Gly Asn Gln Ile Ile Pro Asp Thr Ala Ile Leu
       290                 295                 300
   Ser Val Val Pro Phe His His Gly Phe Gly Met Phe Thr Thr Leu Gly
   305                 310                 315                 320
   Tyr Leu Ile Cys Gly Phe Arg Val Val Leu Met Tyr Arg Phe Glu Glu
                   325                 330                 335
   Glu Leu Phe Leu Arg Ser Leu Gln Asp Tyr Lys Ile Gln Ser Ala Leu
           340                 345                 350
   Leu Val Pro Thr Leu Phe Ser Phe Phe Ala Lys Ser Thr Leu Ile Asp
       355                 360                 365
   Lys Tyr Asp Leu Ser Asn Leu His Glu Ile Ala Ser Gly Gly Ala Pro
       370                 375                 380
   Leu Ser Lys Glu Val Gly Glu Ala Val Ala Lys Arg Phe His Leu Pro
   385                 390                 395                 400
   Gly Ile Arg Gln Gly Tyr Gly Leu Thr Glu Thr Thr Ser Ala Ile Leu
                   405                 410                 415
   Ile Thr Pro Glu Gly Asp Asp Lys Pro Gly Ala Val Gly Lys Val Val
               420                 425                 430
   Pro Phe Phe Glu Ala Lys Val Val Asp Leu Asp Thr Gly Lys Thr Leu
           435                 440                 445
   Gly Val Asn Gln Arg Gly Glu Leu Cys Val Arg Gly Pro Met Ile Met
       450                 455                 460
   Ser Gly Tyr Val Asn Asn Pro Glu Ala Thr Asn Ala Leu Ile Asp Lys
   465                 470                 475                 480
   Asp Gly Trp Leu His Ser Gly Asp Ile Ala Tyr Trp Asp Glu Asp Glu
                   485                 490                 495
   His Phe Phe Ile Val Asp Arg Leu Lys Ser Leu Ile Lys Tyr Lys Gly
               500                 505                 510
   Tyr Gln Val Ala Pro Ala Glu Leu Glu Ser Ile Leu Leu Gln His Pro
           515                 520                 525
   Asn Ile Phe Asp Ala Gly Val Ala Gly Leu Pro Asp Asp Asp Ala Gly
       530                 535                 540
   Glu Leu Pro Ala Ala Val Val Val Leu Glu His Gly Lys Thr Met Thr
   545                 550                 555                 560
   Glu Lys Glu Ile Val Asp Tyr Val Ala Ser Gln Val Thr Thr Ala Lys
                   565                 570                 575
   Lys Leu Arg Gly Gly Val Val Phe Val Asp Glu Val Pro Lys Gly Leu
               580                 585                 590
   Thr Gly Lys Leu Asp Ala Arg Lys Ile Arg Glu Ile Leu Ile Lys Ala
   595                 600                 605
   Lys Lys Gly Gly Lys
   610

<210>  24
<211>  535
<212>  PRT
<213>  Artificial Sequence

<220>
<223>  DED-HRV3C

<400>  24

Met Asp Tyr Lys Asp Asp Asp Asp Lys Gly Met Ala Ser Asp Leu Ser
1               5                   10                  15

Asn Val Glu Gly Lys Thr Gly Asn Ala Thr Asp Glu Glu Glu Glu Glu
            20                  25                  30

Glu Glu Glu Glu Glu Glu Glu Asp Asp Asp Asp Asp Asp Asp Asp Asp
        35                  40                  45

Asp Asp Asp Glu Asp Ser Gly Ala Glu Ile Gln Asp Asp Asp Glu Glu
    50                  55                  60

Gly Phe Asp Asp Glu Glu Glu Phe Asp Asp Asp Asp Asp Asp Glu His
65                  70                  75                  80

Asp Asp Asp Asp Leu Glu Asn Glu Glu Asn Glu Leu Glu Glu Leu Glu
                85                  90                  95

Glu Arg Val Glu Ala Arg Lys Lys Met His His His His His His Ser
            100                 105                 110

Ser Gly Met Ser Pro Ile Leu Gly Tyr Trp Lys Ile Lys Gly Leu Val
        115                 120                 125

Gln Pro Thr Arg Leu Leu Leu Glu Tyr Leu Glu Glu Lys Tyr Glu Glu
    130                 135                 140

His Leu Tyr Glu Arg Asp Glu Gly Asp Lys Trp Arg Asn Lys Lys Phe
145                 150                 155                 160

Glu Leu Gly Leu Glu Phe Pro Asn Leu Pro Tyr Tyr Ile Asp Gly Asp
                165                 170                 175

Val Lys Leu Thr Gln Ser Met Ala Ile Ile Arg Tyr Ile Ala Asp Lys
            180                 185                 190

His Asn Met Leu Gly Gly Cys Pro Lys Glu Arg Ala Glu Ile Ser Met
        195                 200                 205

Leu Glu Gly Ala Val Leu Asp Ile Arg Tyr Gly Val Ser Arg Ile Ala
    210                 215                 220

Tyr Ser Lys Asp Phe Glu Thr Leu Lys Val Asp Phe Leu Ser Lys Leu
225                 230                 235                 240

Pro Glu Met Leu Lys Met Phe Glu Asp Arg Leu Cys His Lys Thr Tyr
                245                 250                 255

Leu Asn Gly Asp His Val Thr His Pro Asp Phe Met Leu Tyr Asp Ala
            260                 265                 270

Leu Asp Val Val Leu Tyr Met Asp Pro Met Cys Leu Asp Ala Phe Pro
        275                 280                 285

Lys Leu Val Cys Phe Lys Lys Arg Ile Glu Ala Ile Pro Gln Ile Asp
    290                 295                 300

Lys Tyr Leu Lys Ser Ser Lys Tyr Ile Ala Trp Pro Leu Gln Gly Trp
305                 310                 315                 320

Gln Ala Thr Phe Gly Gly Gly Asp His Pro Pro Lys Ser Asp Leu Val
                325                 330                 335

Pro Arg Gly Ser Pro Glu Phe Pro Gly Arg Leu Glu Arg Pro His Arg
            340                 345                 350

Asp Gly Pro Asn Thr Glu Phe Ala Leu Ser Leu Leu Arg Lys Asn Ile
        355                 360                 365

Met Thr Ile Thr Thr Ser Lys Gly Glu Phe Thr Gly Leu Gly Ile His
    370                 375                 380

Asp Arg Val Cys Val Ile Pro Thr His Ala Gln Pro Gly Asp Asp Val
385                 390                 395                 400

Leu Val Asn Gly Gln Lys Ile Arg Val Lys Asp Lys Tyr Lys Leu Val
                405                 410                 415

Asp Pro Glu Asn Ile Asn Leu Glu Leu Thr Val Leu Thr Leu Asp Arg
            420                 425                 430

Asn Glu Lys Phe Arg Asp Ile Arg Gly Phe Ile Ser Glu Asp Leu Glu
        435                 440                 445

Gly Val Asp Ala Thr Leu Val Val His Ser Asn Asn Phe Thr Asn Thr
    450                 455                 460

Ile Leu Glu Val Gly Pro Val Thr Met Ala Gly Leu Ile Asn Leu Ser
465                 470                 475                 480

Ser Thr Pro Thr Asn Arg Met Ile Arg Tyr Asp Tyr Ala Thr Lys Thr
                485                 490                 495

Gly Gln Cys Gly Gly Val Leu Cys Ala Thr Gly Lys Ile Phe Gly Ile
            500                 505                 510

His Val Gly Gly Asn Gly Arg Gln Gly Phe Ser Ala Gln Leu Lys Lys
        515                 520                 525

Gln Tyr Phe Val Glu Lys Gln
    530                 535

<210>  25
<211>  78
<212>  DNA
<213>  Artificial Sequence

<220>
<223>  DE12

<400>  25
gatgacaaag gtatggctag cgaggaggaa gaggaagaag atgatgatga tgacgacgct      60

agcctggaag ttctgttc                                                    78

<210>  26
<211>  78
<212>  DNA
<213>  Artificial Sequence

<220>
<223>  DE12

<400>  26
gaacagaact tccaggctag cgtcgtcatc atcatcatct tcttcctctt cctcctcgct      60

agccatacct ttgtcatc                                                    78

<210>  27
<211>  30
<212>  DNA
<213>  Artificial Sequence

<220>
<223>  primer1

<400>  27
cgatacaaat ggaaataata accatctcgc                                        30


<210>  28
<211>  30
<212>  DNA
<213>  Artificial Sequence

<220>
<223>  primer2

<400>  28
cgcacagaat ctaacgctta ataaatgtac                                        30


<210>  29
<211>  825
<212>  DNA
<213>  Artificial Sequence

<220>
<223>  NQ01

<400>  29
atggtgggac gccgtgctct gatcgtgctc gctcactcgg aaagaacatc gttcaactac       60

gctatgaagg aggctgctgc cgctgccctg aagaagaagg gctgggaggt ggtcgaatcc      120

gacttgtacg ctatgaactt caaccccatc atctctcgta aggacatcac cggcaagctg      180

aaggatccag ccaacttcca gtacccggct gagtcagttt tggcctacaa ggaaggccac      240

ctgtcgcctg acatcgtggc tgagcaaaag aagctcgaag ctgccgattt ggttatcttc      300

cagttcccct tgcaatggtt cggtgtgcct gctatcctga agggctggtt cgagagggtc      360

ttcatcggag aattcgccta cacttacgct gccatgtacg acaagggtcc attcagatcg      420

aagaaggccg tcctgtccat caccactggt ggcagcggat caatgtacag cctccaggga      480

atccacggtg acatgaacgt catcctgtgg ccgatccaat ctggcatcct ccacttctgc      540

ggattccagg tgttggagcc acaactgaca tactccatcg gacacacccc agctgacgct      600

cgtatccaga tcctcgaagg atggaagaag cgcttggaga acatctggga cgaaactccc      660

ttgtacttcg ctccttcctc tctgttcgat ctcaacttcc aggccggttt cctcatgaag      720

aaggaggtcc aagacgagga aaagaacaag aagttcggcc tgtctgttgg acaccacctc      780

ggcaagagca tccccacaga taaccagatc aaggctagga agtaa                      825


<210>  30
<211>  30
<212>  DNA
<213>  Artificial Sequence

<220>

<223>  primer3F

<400>  30
ctgttccagg gtcccatggt gggacgccgt                                        30


<210>  31
<211>  36
<212>  DNA
<213>  Artificial Sequence

<220>
<223>  primer3R

<400>  31
ggagctcgaa ttcccttact tcctagcctt gatctg                                 36


<210>  32
<211>  96
<212>  DNA
<213>  Artificial Sequence

<220>
<223>  DE18

<400>  32
gatgacaaag gtatggctag cgaagaagag gaggaggaag aggaagaaga tgatgatgat       60

gacgacgacg acgacgctag cctggaagtt ctgttc                                 96


<210>  33
<211>  96
<212>  DNA
<213>  Artificial Sequence

<220>
<223>  DE18

<400>  33
gaacagaact tccaggctag cgtcgtcgtc gtcgtcatca tcatcatctt cttcctcttc       60

ctcctcctct tcttcgctag ccataccttt gtcatc                                 96


<210>  34
<211>  273
<212>  DNA
<213>  Artificial Sequence

<220>
<223>  DED

<400>  34
gatctaagta atgtggaagg taagacagga aatgcaacag atgaagagga ggaagaagag       60

gaggaggaag aggaagaaga tgatgatgat gacgacgacg acgacgatga tgatgaagac      120

tctggagctg agatacaaga tgatgatgag gaaggttttg atgatgaaga ggaatttgat      180

gatgacgatg atgatgaaca tgatgatgat gatcttgaga atgaggaaaa cgaactggaa      240

gagttggaag agagggtaga agccaggaag aaa 273

<210> 35
<211> 34
<212> DNA
<213> Artificial Sequence

<220>
<223> primer4F

<400> 35
aatagatctt ggtacccatg gaagaggagg aaga 34

<210> 36
<211> 63
<212> DNA
<213> Artificial Sequence

<220>
<223> primer4R

<400> 36
ggagctcgaa ttcccgggac cctggaacag aacttccagg gtacctttat catcatcgtc 60

gtc 63

<210> 37
<211> 132
<212> DNA
<213> Artificial Sequence

<220>
<223> DE30

<400> 37
gaacagaact tccaggctag catcatcatc gtcgtcgtcg tcgtcatcat catcatcgtc 60

gtcgtcctct tcctcttctt cctcttcctc ctcctcttct tcctcctctt cgctagccat 120

acctttgtca tc 132

<210> 38
<211> 132
<212> DNA
<213> Artificial Sequence

<220>
<223> DE30

<400> 38
gatgacaaag gtatggctag cgaagaggag gaagaagagg aggaggaaga ggaagaagag 60

gaagaggacg acgacgatga tgatgatgac gacgacgacg acgatgatga tgctagcctg 120

gaagttctgt tc 132

<210> 39
<211> 150

<212>  DNA
<213>  Artificial Sequence

<220>
<223>  DE36

<400>  39
gaacagaact tccaggctag catcatcatc gtcgtcgtcg tcgtcatcat catcatcgtc      60

gtcgtcgtcg tcgtcctctt cctcctcttc ctcttcttcc tcttcctcct cctcttcttc     120

ctcctcttcg ctagccatac ctttgtcatc                                      150


<210>  40
<211>  150
<212>  DNA
<213>  Artificial Sequence

<220>
<223>  DE36

<400>  40
gatgacaaag gtatggctag cgaagaggag gaagaagagg aggaggaaga ggaagaagag      60

gaagaggagg aagaggacga cgacgacgac gacgatgatg atgatgacga cgacgacgac     120

gatgatgatg ctagcctgga agttctgttc                                      150


<210>  41
<211>  36
<212>  DNA
<213>  Artificial Sequence

<220>
<223>  primer5F

<400>  41
caaaggtatg gctagcgatc taagtaatgt ggaagg                                36


<210>  42
<211>  35
<212>  DNA
<213>  Artificial Sequence

<220>
<223>  primer5R

<400>  42
gaacttccag gctagctttc ttcctggctt ctacc                                 35


<210>  43
<211>  38
<212>  DNA
<213>  Artificial Sequence

<220>
<223>  primer6F

<400>  43

gacaaaggta tggctagcga tctaagtaat gtggaagg 38

<210> 44
<211> 37
<212> DNA
<213> Artificial Sequence

<220>
<223> primer6R

<400> 44
ggaacagaac ttccaggcta gcctcagctc cagagtc 37

<210> 45
<211> 114
<212> DNA
<213> Artificial Sequence

<220>
<223> EO24

<400> 45
gatgacaaag gtatggctag cgaagaggag gaagaagagg aggaggaaga ggaagaagaa 60

gaggaggaag aagaggagga ggaagaggaa gaagctagcc tggaagttct gttc 114

<210> 46
<211> 114
<212> DNA
<213> Artificial Sequence

<220>
<223> EO24

<400> 46
gaacagaact tccaggctag cttcttcctc ttcctcctcc tcttcttcct cctcttcttc 60

ttcctcttcc tcctcctctt cttcctcctc ttcgctagcc atacctttgt catc 114

<210> 47
<211> 35
<212> DNA
<213> Artificial Sequence

<220>
<223> primer7F

<400> 47
gttccagggt ccagctagcg atctaagtaa tgtgg 35

<210> 48
<211> 34
<212> DNA
<213> Artificial Sequence

<220>
<223> primer7R

<400>  48
cgtccttgta gtcgctagct ttcttcctgg cttc                                    34


<210>  49
<211>  33
<212>  DNA
<213>  Artificial Sequence

<220>
<223>  primer8F

<400>  49
cctataaata gatctcccat ggtgggacgc cgt                                     33


<210>  50
<211>  36
<212>  DNA
<213>  Artificial Sequence

<220>
<223>  primer8R

<400>  50
gaacagaact tccagcccct tcctagcctt gatctg                                  36


<210>  51
<211>  39
<212>  DNA
<213>  Artificial Sequence

<220>
<223>  primer9F

<400>  51
gttccagggt ccagctagcg atctaagtaa tgtggaagg                               39


<210>  52
<211>  37
<212>  DNA
<213>  Artificial Sequence

<220>
<223>  primer9R

<400>  52
catcgtcctt gtagtcgcta gcctcagctc cagagtc                                 37


<210>  53
<211>  1638
<212>  DNA
<213>  Artificial Sequence

<220>
<223>  Luciferase

<400>  53
gaagacgcca aaaacataaa gaaaggcccg gcgccattct atcctctaga ggatggaacc        60

gctggagagc aactgcataa ggctatgaag agatacgccc tggttcctgg aacaattgct 120

tttacagatg cacatatcga ggtgaacatc acgtacgcgg aatacttcga aatgtccgtt 180

cggttggcag aagctatgaa acgatatggg ctgaatacaa atcacagaat cgtcgtatgc 240

agtgaaaact ctcttcaatt ctttatgccg gtgttgggcg cgttatttat cggagttgca 300

gttgcgcccg cgaacgacat ttataatgaa cgtgaattgc tcaacagtat gaacatttcg 360

cagcctaccg tagtgtttgt ttccaaaaag gggttgcaaa aaattttgaa cgtgcaaaaa 420

aaattaccaa taatccagaa aattattatc atggattcta aaacggatta ccagggattt 480

cagtcgatgt acacgttcgt cacatctcat ctacctcccg gttttaatga atacgatttt 540

gtaccagagt cctttgatcg tgacaaaaca attgcactga taatgaattc ctctggatct 600

actgggttac ctaagggtgt ggcccttccg catagaactg cctgcgtcag attctcgcat 660

gccagagatc ctatttttgg caatcaaatc attccggata ctgcgatttt aagtgttgtt 720

ccattccatc acggttttgg aatgtttact acactcggat atttgatatg tggatttcga 780

gtcgtcttaa tgtatagatt tgaagaagag ctgtttttac gatcccttca ggattacaaa 840

attcaaagtg cgttgctagt accaacccta ttttcattct tcgccaaaag cactctgatt 900

gacaaatacg atttatctaa tttacacgaa attgcttctg ggggcgcacc tctttcgaaa 960

gaagtcgggg aagcggttgc aaaacgcttc catcttccag ggatacgaca aggatatggg 1020

ctcactgaga ctacatcagc tattctgatt acacccgagg gggatgataa accgggcgcg 1080

gtcggtaaag ttgttccatt ttttgaagcg aaggttgtgg atctggatac cgggaaaacg 1140

ctgggcgtta atcagagagg cgaattatgt gtcagaggac ctatgattat gtccggttat 1200

gtaaacaatc cggaagcgac caacgccttg attgacaagg atggatggct acattctgga 1260

gacatagctt actgggacga agacgaacac ttcttcatag ttgaccgctt gaagtcttta 1320

attaaataca aaggatatca ggtggccccc gctgaattgg aatcgatatt gttacaacac 1380

cccaacatct tcgacgcggg cgtggcaggt cttcccgacg atgacgccgg tgaacttccc 1440

gccgccgttg ttgttttgga gcacggaaag acgatgacgg aaaaagagat cgtggattac 1500

gtcgccagtc aagtaacaac cgcgaaaaag ttgcgcggag gagttgtgtt tgtggacgaa 1560

gtaccgaaag gtcttaccgg aaaactcgac gcaagaaaaa tcagagagat cctcataaag 1620

gccaagaagg gcggaaag 1638

<210> 54
<211> 30
<212> DNA
<213> Artificial Sequence

<220>
<223> primer10F

<400>  54
ctgttccagg gtcccgaaga cgccaaaaac                                        30


<210>  55
<211>  35
<212>  DNA
<213>  Artificial Sequence

<220>
<223>  primer10R

<400>  55
ggagctcgaa ttcccttact ttccgccctt cttgg                                  35


<210>  56
<211>  1296
<212>  DNA
<213>  Artificial Sequence

<220>
<223>  HRV3C

<400>  56
atgcaccacc accatcatca ttcgagcggt atgagcccca ttttggggta ttggaaaatc      60

aaaggtctgg ttcaaccaac ccggctcctg cttgaatatc ttgaagagaa atacgaagag     120

catctgtatg aacgtgacga aggcgataaa tggcgcaata agaagtttga acttggcctg     180

gagtttccga acttgccgta ttacattgat ggcgatgtga aactgacaca gtctatggcg     240

attattcgct atattgcgga caaacacaac atgttaggcg gttgcccgaa agaacgtgcg     300

gaaatctcaa tgttagaagg ggctgttctc gatattcgct atggcgtgtc tcgtatcgca     360

tacagtaaag actttgaaac gctgaaagtc gattttcttt cgaaattgcc ggagatgctg     420

aaaatgttcg aagatcggtt gtgccacaaa acgtatctga acggggatca tgtcacccat     480

ccggatttca tgttgtacga tgctctggat gtggtgctgt atatggaccc aatgtgcttg     540

gacgcgtttc caaagctggt gtgtttcaag aaacgcattg aggccattcc gcagattgat     600

aaatacctga aaagctcgaa atatattgcg tggcctctgc agggttggca agccaccttt     660

ggtggcggag atcaccctcc gaaaagcgat ctggtcccgc gtgggagtcc tgaatttcca     720

ggtcgccttg agcgcccgca tcgtgatggt ccgaacacgg aattcgcact gtccctcctg     780

cgcaagaaca ttatgacaat caccacgagc aaaggcgaat tcactggact gggaatccat     840

gatcgcgtgt gtgttattcc cacccatgca cagcctggtg atgacgtcct ggtaaatggc     900

cagaaaatcc gcgttaaaga caaatacaaa ctggtagacc cggaaaacat caatctcgaa     960

ctgaccgtgt taaccttaga ccgtaacgag aaatttcgcg acattcgcgg tttcatttcc    1020

gaggacctcg aaggtgtgga tgcaacgctg gtagtgcatt ccaacaattt cacgaatacc    1080

atcctggaag ttggcccggt tacaatggcc ggcttaatca acctgtctag tactcccacc    1140

aatcgtatga ttcgctatga ttacgcgacc aagactggcc aatgtggtgg agtcttatgc     1200

gctactggca aaatctttgg gatccacgtt ggtggcaatg gccgtcaggg cttttcagcc     1260

caactgaaga aacagtactt cgtagaaaag cagtaa                               1296


<210>  57
<211>  42
<212>  DNA
<213>  Artificial Sequence

<220>
<223>  primer11F

<400>  57
cgatgacaaa ggtatggcta gcgatctaag taatgtggaa gg                        42


<210>  58
<211>  39
<212>  DNA
<213>  Artificial Sequence

<220>
<223>  primer11R

<400>  58
gatgatggtg gtggtgcatt ttcttcctgg cttctaccc                            39


<210>  59
<211>  39
<212>  DNA
<213>  Artificial Sequence

<220>
<223>  primer12F

<400>  59
gggtagaagc caggaagaaa atgcaccacc accatcatc                            39


<210>  60
<211>  44
<212>  DNA
<213>  Artificial Sequence

<220>
<223>  primer12R

<400>  60
ctcgaggagc tcgaattccc ttactgcttt tctacgaagt actg                      44


<210>  61
<211>  45
<212>  DNA
<213>  Artificial Sequence

<220>
<223>  primer13F

<400>  61
ctttaagaag gagatataca tatggactac aaggacgacg atgac                    45


<210>  62
<211>  45
<212>  DNA
<213>  Artificial Sequence

<220>
<223>  primer13R

<400>  62
ccaccagtca tgctagccat atgttactgc ttttctacga agtac                    45


<210>  63
<211>  16
<212>  DNA
<213>  Artificial Sequence

<220>
<223>  primer14

<400>  63
atgcgtccgg cgtaga                                                    16


<210>  64
<211>  18
<212>  DNA
<213>  Artificial Sequence

<220>
<223>  primer15

<400>  64
ccctcaagac ccgtttag                                                  18


<210>  65
<211>  6
<212>  PRT
<213>  Artificial Sequence

<220>
<223>  DE6

<400>  65

Glu Glu Glu Asp Asp Asp
1               5


<210>  66
<211>  60
<212>  DNA
<213>  Artificial Sequence

<220>
<223>  DE6

<400>  66

gatgacaaag gtatggctag cgaggaagaa gatgatgatg ctagcctgga agttctgttc 60

<210> 67
<211> 60
<212> DNA
<213> Artificial Sequence

<220>
<223> DE6

<400> 67
gaacagaact tccaggctag catcatcatc ttcttcctcg ctagccatac ctttgtcatc 60

## Description

### TECHNICAL FIELD

The present invention relates to a method for purifying a protein, a fusion protein containing a peptide tag, and a production method thereof.

### BACKGROUND

According to the progress of molecular biology, a system for mass expressing an objective protein by introducing a recombinant gene encoding the objective protein into a host such as E. coli, a yeast or a cell has been developed. In such system, a sample prepared from the host contains contaminant proteins such as host-derived endogenous proteins, together with the objective protein. Thus, the objective protein needs to be further purified. As a method for purifying an objective protein, for example, a method for purifying VP1 protein by introducing eight glutamic acids and cysteine into polyoma VP1 protein is disclosed in Stubenrauch K, et al, Purification of a viral coat protein by an engineered polyionic sequence. J. Chromatogr. B. Biomed. Sci. Appl: 737: 77-84, 2000.

### SUMMARY OF THE INVENTION

An object of the present invention is to provide a purification method capable of easily obtaining high purity recombinant protein.

One aspect of the present invention is a method for purifying a protein, comprising steps of: preparing a sample comprising a fusion protein and contaminant proteins, the fusion protein comprising an amino acid sequence of a peptide tag and an amino acid sequence of a target protein, and separating the contaminant proteins and the fusion protein, wherein the peptide tag comprises 12 or more acidic amino acid residues.

In particular embodiments, the peptide tag comprises any of the amino acid sequences of SEQ ID NOs. 1 to 8.

In particular embodiments, the peptide tag comprises 18 or more acidic amino acid residues.

In particular embodiments, the isoelectric point of the fusion protein is less than 6.

In particular embodiments, the salt concentration of the sample is 50 mM or more and 500 mM or less.

In particular embodiments, in the sample preparation step, a vector containing a polynucleotide encoding the fusion protein is introduced into a host cell, and the protein is expressed in the host cell to prepare the sample.

In particular embodiments, the fusion protein further comprises a cleavable site recognized by a protease, between the amino acid sequence of a peptide tag and the amino acid sequence of the target protein.

In particular embodiments, in the step of separating the contaminant proteins, the fusion protein and the contaminant proteins are separated using an ion exchange resin.

In particular embodiments, the ion exchange resin is an anion exchange resin.

In particular embodiments, the step of separating the contaminant proteins comprises passing the sample through an anion exchange resin to obtain (i) the anion exchange resin to which the fusion protein is bound and (ii) a flow-through fraction comprising the contaminant proteins, and eluting the fusion protein from the anion exchange resin using a buffer with a salt concentration of 600 mM or more.

In particular embodiments, after the step of separating the contaminant proteins, the method further comprises a step of separating the fusion protein into the target protein and the peptide tag in a solution, using the protease recognizing the cleavable site.

In particular embodiments, in the step of separating the fusion protein, the solution comprising the separated target protein and the separated peptide tag is passed through an anion exchange resin, and (i) the anion exchange resin to which the separated peptide tag is bound, and (ii) a flow-through fraction comprising the target protein, are obtained.

In particular embodiments, the salt concentration of the solution comprising the target protein and the peptide tag is 500 mM or less.

Another aspect of the present invention is a fusion protein comprising an amino acid sequence of a peptide tag and an amino acid sequence of a target protein, the peptide tag comprising 12 or more acidic amino acid residues.

In particular embodiment, the peptide tag comprises any of the amino acid sequences of SEQ ID NOs. 1 to 8.

In particular embodiments, the peptide tag comprises 18 or more acidic amino acid residues.

In particular embodiments, the isoelectric point of the fusion protein is less than 6.

In particular embodiments, the fusion protein further comprises a cleavable site recognized by a protease, between the amino acid sequence of the peptide tag and the amino acid sequence of the target protein.

Another aspect of the present invention is a method for producing the fusion protein, comprising steps of introducing a polynucleotide encoding the amino acid sequence of the peptide tag and a polynucleotide encoding the amino acid sequence of the target protein into a host cell, and expressing the fusion protein in the host cell.

Another aspect of the present invention is a method for producing a pure target protein, the method comprising producing the fusion protein as described above, purifying the fusion protein by separating the contaminant proteins and the fusion protein and, after said purification, cleaving said fusion protein to obtain said target protein and separating said target protein from said peptide tag and optionally said enzyme used for said cleaving.

Another aspect of the present invention is use of a peptide tag comprising 12 or more acidic amino acid residues, for the above-mentioned method for purifying a protein.

A higher purity recombinant protein can be obtained by a simple method.

### BRIEF DESCRIPTION OF THE DRAWINGS

Figs. 1A to 1C are diagrams schematically showing a principle of the purification methods provided herein, in which Fig. 1A shows a separation step, and Figs. 1B and 1C show a target protein acquisition step.
Fig. 2 is a figure showing the results of SDS (Sodium dodecyl sulfate)-polyacrylamide gel electrophoresis (SDS-PAGE) in Example 1.
Fig. 3 is a figure showing the results of SDS-PAGE in Example 2.
Fig. 4 is a figure showing the results of SDS-PAGE in Example 3.
Fig. 5 is a figure showing the results of SDS-PAGE in Example 4.
Fig. 6 is a figure showing the results of SDS-PAGE in Example 5.
Fig. 7 is a figure showing the results of SDS-PAGE in Example 6.
Fig. 8 is a figure showing the results of SDS-PAGE in Example 7.
Fig. 9 is a figure showing the results of SDS-PAGE in Example 8.
Fig. 10 is a figure showing the results of SDS-PAGE in Example 9.
Fig. 11 is a figure showing the results of SDS-PAGE in Example 10.
Fig. 12 is a figure showing the results of SDS-PAGE in Example 11.
Fig. 13 is a figure showing the results of SDS-PAGE in Example 12.
Fig. 14 is a figure showing the results of SDS-PAGE in Example 13.
Fig. 15 is a figure showing the results of SDS-PAGE in Comparative Example 1.
Fig. 16 is a figure showing the results of SDS-PAGE in Example 14.
Fig. 17 is a figure showing the results of SDS-PAGE in Example 15.
Fig. 18 is a figure showing the results of SDS-PAGE in Example 16.
Fig. 19 is a figure showing the results of SDS-PAGE in Example 17.
Fig. 20 is a figure showing the results of SDS-PAGE in Example 18.
Fig. 21 is a figure showing the results of SDS-PAGE in Example 19.
Fig. 22 is a figure showing the results of SDS-PAGE in Example 20.
Fig. 23 is a figure showing the results of SDS-PAGE in Example 21.
Fig. 24 is a figure showing the results of SDS-PAGE in Example 22.
Fig. 25 is a figure showing the results of SDS-PAGE in Example 23.
Fig. 26 is a figure showing the results of SDS-PAGE in Example 24.
Fig. 27 is a figure showing the results of SDS-PAGE in Example 25.
Fig. 28 is a figure showing the results of SDS-PAGE in Example 26.
Fig. 29 is a figure showing the results of SDS-PAGE in Example 27.
Fig. 30 is a figure showing the results of SDS-PAGE in Example 28.

### DETAILED DESCRIPTION OF THE PREFERRED EMBODIMENTS

The purification methods of the present invention include steps of preparing a sample containing a fusion protein containing an amino acid sequence of a peptide tag and an amino acid sequence of a target protein and contaminant proteins (sample preparation step), and separating the fusion protein and the contaminant proteins (separation step). The contaminant protein means a protein such as host-derived endogenous proteins, other than the fusion protein and the target protein.

### (Sample Preparation Step)

The fusion protein contains amino acid sequences of a peptide tag and a target protein. The peptide tag may be one containing 12 or more acidic amino acid residues. The number of the acidic amino acid residues in the peptide tag is preferably 18 residues or more, more preferably 24 residues or more, further preferably 30 residues or more, and particularly preferably 36 residues or more, since separation from the contaminant proteins is improved, and an effect on separation due to the isoelectric point of the target protein, pH of the buffer and the like can be more suppressed, in the separation step. The upper limit of the number of the acidic amino acid residues is not particularly limited. The upper limit of the number of the acidic amino acid residues is preferably 100 residues or less, and more preferably 70 residues or less, from the viewpoint of the effect on the three-dimensional structure of the fusion protein and the like. The acidic amino acid residues contained in the peptide tag may be either one of an aspartic acid residue or a glutamic acid residue or both of them. The peptide tag may contain a neutral amino acid residue and/or a basic amino acid residue, other than the acidic amino acid residue. The ratio of the number of the acidic amino acid residues to the number of the amino acid residues of the total peptide tags is preferably 20% or more and more preferably 60% or more, since separation from the contaminant proteins is improved. In the sequence of the total peptide tags, the amino acid sequence containing the acidic amino acid residues may be a sequence in which all acidic amino acid residues are continuous, or one or a plurality of neutral amino acid residues and/or basic amino acid sequences may be interposed between the acidic amino acid residues. In particular embodiments, the peptide comprises at least 4 consecutive acidic amino acid residues. More particularly, the peptide comprises at least 6 consecutive acidic amino acid residues. Preferably the peptide comprises at least 6 consecutive aspartic acid residues and/or at least 6 consecutive glutamic acid residues or at least 12 consecutive acidic amino acids which are preferably selected from aspartic acid and/or glutamic acid. The isoelectric point (pI) of the peptide tag is preferably 5 or less and more preferably 4 or less, since separation from the contaminant proteins is improved, and the effects on separation due to the isoelectric point of the target protein, pH of the buffer and the like can be more suppressed. The isoelectric point is a pH value when the positive and negative ion concentrations in the aqueous solution become equal to each other. The isoelectric point can be measured, for example, by isoelectric electrophoresis, or the like. Specific examples of the peptide tag include the following peptide tags 1 to 8 (D; aspartic acid residue, E; glutamic acid residue).

### <Peptide Tag>

Peptide tag 1 EEEEEEDDDDDD (DE12; SEQ ID NO.: 1)
Peptide tag 2 EEEEEEEEEDDDDDDDDD (DE18; SEQ ID NO.: 2)
Peptide tag 3 EEEEEEEEEEEEDDDDDDDDDDDD (DE24; SEQ ID NO.: 3)
Peptide tag 4 EEEEEEEEEEEEEEEDDDDDDDDDDDDDDD (DE30; SEQ ID NO.: 4)
Peptide tag 5 EEEEEEEEEEEEEEEEEEDDDDDDDDDDDDDDDDDD (DE36; SEQ ID NO.: 5)
Peptide tag 6 
NVEGKTGNATDEEEEEEEEEEEEEEDDDDDDDDDDDDEDSGAEIQDDDEE

GFDDEEEFDDDDDDEHDDDDLENEENELEELEERVEARKK (DED; SEQ ID NO.:

6)
Peptide tag 7 DLSNVEGKTGNATDEEEEEEEEEEEEDDDDDDDDDDDDEDSGAE (DES; SEQ ID NO.: 7)
Peptide tag 8 EEEEEEEEEEEEEEEEEEEEEEEE (EO24; SEQ ID NO.: 8)

The target protein is not particularly limited. Examples of the target protein include proteins such as enzymes, receptors, interferons, interleukins, antibodies, and fluorescent proteins. The isoelectric point of the target protein is not particularly limited. Generally, the isoelectric point of a protein is 6 to 7, and even when any protein is targeted, the isoelectric point can be lowered by fusing a peptide tag, to an extent that the peptide tag can be sufficiently separated from the contaminant proteins.

In the fusion protein, the peptide tag may be bound to either N-terminal side or C-terminal side of the target protein. The peptide tag may be adjacently bound to the target protein, or other amino acid sequence may be inserted between the target protein and the peptide tag. For example, when an amino acid sequence of the cleavable site to be recognized by a protease is inserted between the amino acid sequences of the fusion protein and the peptide tag, it becomes easier to acquire a target protein by cleaving the peptide tag from the fusion protein. The isoelectric point of the fusion protein is not particularly limited. The isoelectric point of the fusion protein is preferably less than 6 and more preferably less than 5. The isoelectric point of many proteins is generally 6 to 7, thus when the isoelectric point of the fusion protein is less than 6, separation of the fusion protein from the contaminant proteins becomes easier, and purity of the fusion protein can be improved.

The sample containing a fusion protein and contaminant proteins can be acquired by introducing a polynucleotide containing a base sequence encoding a peptide tag and a base sequence encoding a target protein into a host cell and expressing the fusion protein in the host cell. Expression of the fusion protein can be performed by a known genetic engineering method. A polynucleotide encoding a peptide tag and a polynucleotide encoding a target protein can be acquired by a known method such as chemical synthesis. The acquired polynucleotides can be amplified according to a known gene amplification method using these as templates. The amplified polynucleotide and an expression vector are treated with a restriction enzyme, and bound using an appropriate DNA ligase, whereby a recombinant expression vector containing the polynucleotides each encoding a peptide tag and a target protein can be constructed. In the construction of the expression vector, the base sequence encoding a peptide tag may be arranged at the 5' side or 3' side of the base sequence encoding a target protein. The base sequence encoding a peptide tag and the base sequence encoding a target protein may be adjacently arranged, or a base sequence encoding a cleavable site recognized by a protease or the like may be inserted there between. The host is not particularly limited. As the host, E. coli, a yeast, an insect cell, an insect body, an animal cell, a plant cell or the like can be used. The expression vector is not particularly limited. The expression vector includes plasmid vectors, phage vectors, virus vectors and the like. The expression vector can be properly selected depending on the used host. The expression vector may contain a regulatory sequence such as a replication origin, a promoter sequence or an enhancer sequence, or a sequence such as a selectable marker. Introduction of an expression vector into a host can be performed according to a known method depending on the host. Examples of the method include a calcium phosphate method, an electroporation method, a lipofection method, and the like. As described above, a transformant in which the expression vector is introduced into the host cell can be obtained. The obtained transformant is incubated under preferred conditions, thus a fusion protein can be produced.

After expressing the fusion protein, treatment such as extraction, crushing, centrifugation, solubilization and body fluid collection is performed on cells of the host, tissue, insect body, and the like, as necessary, to acquire a sample as a cell culture liquid, an extract, a homogenate, a solution, a body fluid, or the like. The sample contains contaminant proteins such as host-derived endogenous proteins, other than the produced fusion protein. For the preparation of the sample, a buffer such as a phosphate buffer, a citrate buffer, an acetate buffer, a tris buffer, a MOPS buffer or a HEPES buffer can be used. These buffers contain a salt with a weak acid or weak base used for obtaining a buffer action. In addition, salts such as sodium chloride, potassium chloride and calcium chloride can be used. The salt concentration of a buffer or sample herein means a concentration of a salt other than the salt intended for buffer action described above. The salt concentration of a sample is preferably 50 mM or more and 500 mM or less, and more preferably 50 mM or more and 250 mM or less, from the viewpoint of stability of the fusion protein, easiness of adjustment in the separation step, and the like. The pH of a sample is preferably 5 or more and 9 or less, and more preferably 6 or more and 8 or less, from the viewpoint of stability of the fusion protein, easiness of adjustment in the separation step, and the like.

When a sample is acquired using an Escherichia coli expression system, a plasmid vector can be used as an expression vector. Specific examples of the plasmid vector include pET, pGEX, pCold, pMAL, pCAL, and the like. The expression vector may contain a promoter sequence such as a replication origin, lac, T7 or tac, an enhancer sequence, or a sequence of a selectable marker such as ampicillin resistance gene or kanamycin resistance gene. Introduction of an expression vector into E. coli can be performed by a calcium phosphate method, an electroporation method, a lipofection method, and the like. After the introduction of an expression vector, E. coli is cultured in a medium containing the selectable marker, whereby a transformant in which the expression vector is introduced into E. coli can be selected. The thus-obtained transformant is proliferated under preferred conditions. Then, the proliferated transformant is expressed and induced depending on the selected promoter to produce a fusion protein. After expressing the fusion protein, a sample containing the fusion protein and contaminant proteins derived from E. coli can be acquired by crushing E. coli or the like.

When using an insect body such as silkworm or insect cells as an expression system, a transfer vector can be used. The transfer vector is transduced into the insect cells together with baculovirus DNA, and then an objective gene can be inserted into the baculovirus DNA by homologous recombination. Examples of the transfer vector include pM01, pM02, pHS01, pHS02, and the like. The transfer vector preferably contains a promoter such as a polyhedrin promoter or a p10 promoter. Examples of the insect cells include Sf9, Sf21, High5, TN-368, Bm5, and the like. Examples of the baculovirus include nuclear polyhedrosis virus (NPV), and specifically, AcMNPV, BmNPV and the like can be exemplified. The transfer vector and linearized baculovirus DNA are introduced into the insect cells, whereby homologous recombination occurs, and a recombinant baculovirus into which an objective gene is inserted is obtained. A virus solution containing a recombinant baculovirus is inoculated into an insect body or insect cells, or the like, whereby the recombinant baculovirus can be infected. The insect body such as silkworm may be in any form of imago, pupa, and larva. The insect body or insect cells are infected with the recombinant baculovirus, and bred or cultured for 1 to 7 days, whereby a fusion protein can be expressed. After expressing the fusion protein, a sample containing the fusion protein and contaminant proteins derived from the insect body or insect cells can be acquired by crushing the insect body or insect cells, or the like.

### (Fusion Protein)

The fusion protein in the methods provided herein can be obtained, as the sample preparation step described above, by a production method including steps of introducing a polynucleotide containing a base sequence encoding an amino acid sequence of a peptide tag and a base sequence encoding an amino acid sequence of a fusion protein into a host cell, and expressing the fusion protein in the host cell. The fusion protein contains the amino acid sequence of a peptide tag containing 12 or more acidic amino acid residues and the amino acid sequence of a target protein. Examples of the fusion protein of the present invention include the following constructs 1 to 12 obtained by fusing NAD(P)H dehydrogenase, quinone 1 human (NQO1; pI = 8.91; SEQ ID NO.: 9) or Luciferase (pI = 6.71; SEQ ID NO.: 10) that is a target protein and the above peptide tags 1 to 8, and inserting a cleavable site recognized by protease HRV3C there between. The following construct 13 obtained by fusing HRV3C (pI = 8.46; SEQ ID NO.: 11) that is a target protein and the above peptide tag 7 is also exemplified.

| | |
|---|---|
| Construct 1 | DE12-HRV3Csite-NQO1 (SEQ ID NO.: 12) |
| Construct 2 | DE18-HRV3Csite-NQO1 (SEQ ID NO.: 13) |
| Construct 3 | DE24-HRV3Csite-NQO1 (SEQ ID NO.: 14) |
| Construct 4 | DE30-HRV3Csite-NQO1 (SEQ ID NO.: 15) |
| Construct 5 | DE36-HRV3Csite-NQO1 (SEQ ID NO.: 16) |
| Construct 6 | DED-HRV3Csite-NQO1 (SEQ ID NO.: 17) |
| Construct 7 | DES-HRV3Csite-NQO1 (SEQ ID NO.: 18) |
| Construct 8 | EO24-HRV3Csite-NQO1 (SEQ ID NO.: 19) |
| Construct 9 | NQO1-HRV3Csite-DED (SEQ ID NO.: 20) |
| Construct 10 | NQO1-HRV3Csite-DES (SEQ ID NO.: 21) |
| Construct 11 | DED-HRV3Csite-Luciferase (SEQ ID NO.: 22) |
| Construct 12 | DES-HRV3Csite-Luciferase (SEQ ID NO.: 23) |
| Construct 13 | DED-HRV3C (SEQ ID NO.: 24) |

### (Separation Step)

As to the sample containing the fusion protein obtained in the sample preparation step and the contaminant proteins, in the methods provided herein, the fusion protein and the contaminant proteins are separated. The phrase "the fusion protein and the contaminant proteins are separated" includes extracting and purifying the fusion protein, and storing into a container different from the contaminant proteins. The contaminant proteins may be substantially completely separated, or a part of the contaminant proteins may be separated. A part or all of the contaminant proteins is separated, whereby the purity of the fusion protein can be improved.

The separation means is not particularly limited, and a known separation means can be used. Separation by an ion exchange resin is preferred, and separation by an anion exchange resin is more preferred, since good separation from the contaminant proteins is obtained. The ion exchange group of the anion exchange resin is not particularly limited, and a quaternary ammonium (QA), a quaternary aminoethyl (QAE), diethylaminoethyl (DEAE) and the like can be used. Specific examples include Hitrap HP Q (GE Healthcare), TOYOPEARL GigaCap Q-650M, TOYOPEARL Q-600C AR, TOYOPEARL QAE-550, TOYOPEARL DEAE-650M, TOYOPEARL SuperQ-650M (all, Tosoh Corporation), Q101, DE101 (Mitsubishi Chemical Corporation), and the like. As a carrier of the ion exchange group, cellulose, dextran, agarose, a hydrophilic vinyl polymer or the like can be used. The sample containing the fusion protein obtained in the sample preparation step and the contaminant proteins is passed through such anion exchange resin to bind the fusion protein to the anion exchange resin. Thereafter, for example, the salt concentration of an eluate is increased to elute the contaminant proteins, then the fusion protein is eluted from the ion exchange resin with the eluate with a high salt concentration, whereby a purified fusion protein can be acquired. Examples of a method for increasing the salt concentration of an eluate include a stepwise method in which the salt concentration is stepwisely changed to elute an objective protein, a gradient method in which the salt concentration is continuously changed to elute an objective protein, and the like. As a buffer used for elution, a buffer such as a phosphate buffer, a citrate buffer, an acetate buffer, a tris buffer, a MOPS buffer or a HEPES buffer can be used. The salt concentration of the buffer is preferably set in the range of 50 mM or more and 2000 mM or less, and more preferably in the range of 50 mM or more and 1000 mM or less, from the viewpoint of separation from the contaminant proteins, stability of the fusion protein, and the like. Particularly, when the salt concentration of the buffer is set at 600 mM or more and more preferably 750 mM or more, separation from the contaminant proteins is improved. The range of pH of the buffer is not particularly limited. The range of pH of the buffer is preferably in the range of 5 or more and 9 or less, and more preferably in the range of 6 or more and 8 or less, from the viewpoint of separation from the contaminant proteins, stability of the fusion protein, and the like.

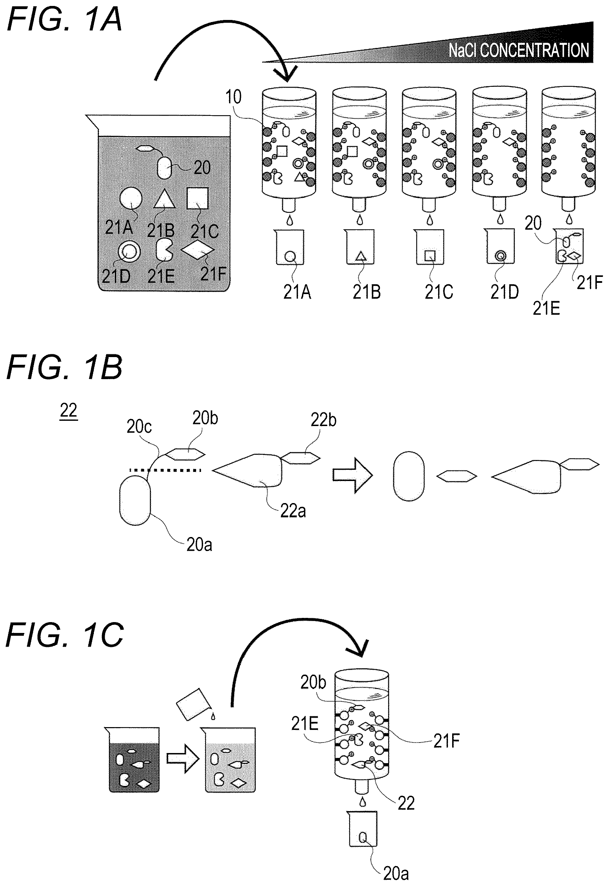

Figs. 1A to 1C are schematic diagrams showing one embodiment, and Fig. 1A is a diagram showing a separation step. The principle of the separation step in the purification method of the present methods will be described based on this diagram. When a fusion protein is expressed in a host cell, a sample such as a disrupted cell suspension contains contaminant proteins 21A to 21F such as host-derived endogenous proteins, other than a fusion protein 20. The isoelectric points of the contaminant proteins 21 are high in the order of A to F. In a particular embodiment, a peptide tag 20b containing 12 or more acidic amino acid residues is bound to a target protein 20a to lower the isoelectric point of the fusion protein 20. The fusion protein 20 in which the isoelectric point is lowered can be held in an anion exchange resin 10 up to a high salt concentration. On the other hand, most of host-derived contaminant proteins cannot be held in the anion exchange resin 10 up to the same salt concentration. The fusion protein and the contaminant proteins are separated, based on the difference in the isoelectric points. Specifically, when a sample is brought into contact with the anion exchange resin 10, the contaminant protein 21A with a high isoelectric point passes without binding to the anion exchange resin. Other contaminant proteins and the fusion protein 20 bind to the anion exchange resin 10. When the salt concentration of the eluate is increased, contaminant proteins are eluted in the higher order of the isoelectric point (21B to 21D). The fusion protein 20 is eluted by an eluate with further higher salt concentration. This elution fraction contains contaminant proteins 21E and 21F with an isoelectric point close to that of the fusion protein 20. The salt concentration of the eluate is adjusted as described above, whereby many contaminant proteins can be separated, thus a high purity fusion protein can be obtained. The difference in the isoelectric points between the fusion protein and the contaminant proteins is relatively large, thus good separation is obtained also by a stepwise method in which the salt concentration is stepwisely changed to elute a protein.

### (Target Protein Acquisition Step)

The fusion protein and the contaminant proteins contained in the sample can be separated as described above. When the fusion protein further contains a cleavable site to be recognized by a protease, the protease is reacted with the separated fusion protein, whereby a peptide tag is cleaved from the fusion protein, and only a target protein can be acquired. The purification methods provided herein, after the separation step, may further include a step of cleaving the peptide tag from the fusion protein in the solution, using a protease as described above, to acquire a target protein.

The kind of the protease is not particularly limited. Examples include HRV3C, PreScission protease, factor Xa, thrombin, TEV protease, and the like. For example, such protease is added to the eluate containing a target protein obtained in the separation step, whereby the peptide tag can be cleaved from the fusion protein. The reaction temperature and the reaction time can be properly set according to the kind of the protease and the like. In the solution after reaction, the target protein, the peptide tag and the protease are contained. The target protein can be separated from this solution, using a known separation means. The separation means is not particularly limited, and a known separation means such as various chromatography can be used. In particular embodiments, the separation step is based on the difference in the isoelectric points between the target protein and the peptide tag when the peptide tag is cleaved from the fusion protein. More particularly separation by an ion exchange resin using the difference in the isoelectric points is preferred, and separation by an anion exchange resin is further preferred. When a solution containing the target protein, the peptide tag and the protease after reaction by the protease is brought into contact with the anion exchange resin, the cleaved peptide tag binds to the ion exchange resin, and the target protein passes without binding to the ion exchange resin. Therefore, the target protein can be acquired by collecting this flow-through fraction. The salt concentration of the solution containing the target protein, the peptide tag and the protease is preferably 500 mM or less and more preferably 300 mM or less, since separation from the peptide tag is improved. The pH of the solution is preferably 5 or more and 9 or less, and more preferably 6 or more and 8 or less. As a buffer used for adjustments of the salt concentration and pH of the solution, a phosphate buffer, a citrate buffer, an acetate buffer, a tris buffer, a MOPS buffer, a HEPES buffer or the like can be used. Even when contaminant proteins remain in the solution fraction containing a fusion protein obtained in the separation step, the isoelectric point of those contaminant proteins is relatively close to that of the fusion protein. Therefore, the difference in the isoelectric point with the remaining contaminant proteins is also increased by cleaving the peptide tag from the fusion protein. Therefore, when using an ion exchange resin, separation from contaminant proteins is also improved, and a higher purity target protein can be obtained. The protease can be separated by a known separation means. When a difference is present in the isoelectric points of the target protein and the protease, both can be easily separated by an ion exchange resin.

Accordingly, in particular embodiments of the methods provided herein, the isoelectric point of the protease is preferably different from the isoelectric point of the target protein, preferably lower than that of the target protein. This makes it possible to easily separate the target protein and the protease, after cleaving the peptide tag with the protease. In particular embodiments this difference in isoelectric point is obtained by fusing the protease with a peptide tag. This makes it possible to adjust the isoelectric point of the protease to a desired value. As the peptide tag fused to the protease, the same one as the peptide tag to be bound to the fusion protein described above can be used. The amino acid sequence of the peptide tag to be bound to the protease may be the same as or different from the amino acid sequence of the peptide tag to be bound to the fusion protein. The amino acid sequence of the peptide tag to be bound to the protease is preferably the same as the amino acid sequence of the peptide tag contained in the fusion protein. Thereby, the protease has the isoelectric point almost same as that of the fusion protein and also different from that of the target protein. The protease fused with the peptide tag preferably does not have a cleavable site to be recognized by this protease, in order to avoid decomposition by itself. When the protease fused with the peptide tag is used, the target protein, the cleaved peptide tag and the protease fused with the peptide tag are contained in the solution after reaction. This solution is passed through an anion exchange resin, whereby the protease fused with the peptide tag also binds to the anion exchange resin, together with the cleaved peptide tag, thus a step of separating the protease is not separately required.

The protease fused with the peptide tag can be obtained by using the protease as the target protein, in the expression of the fusion protein described above. The fusion protein and the protease can be expressed by introducing a vector into which a polynucleotide containing a base sequence encoding both amino acid sequences into a host. A vector into which a polynucleotide encoding the fusion protein is introduced and a vector into which a polynucleotide encoding the protease is inserted may be introduced into the host to express the fusion protein and the protease. The vector into which a polynucleotide encoding the fusion protein is inserted is introduced into the host, and the vector into which a polynucleotide encoding the protease is inserted is introduced into the host, and the fusion protein and the protease are expressed alone respectively, and then may be mixed. In this case, the hosts may be the same kind or the different kind, and are preferably the same kind, in the aspect of easiness of handling after expression and the like. The isoelectric point of the protease fused with the peptide tag is not particularly limited. The isoelectric point is preferably less than 6 and more preferably less than 5, since separation from the target protein is improved.

Based on the Figs. 1B and 1C, the principle of the target protein acquisition step in the purification methods provided herein will be described. The fusion protein 20 contains a cleavable site 20c to be recognized by the protease 22, between the target protein 20a and the peptide tag 20b. In a protease 22 fused with the peptide tag, a peptide tag 22b is bound to a protease 22a. When the fusion protein 20 and the protease 22 are reacted in the solution, the peptide tag 20b is cleaved from the fusion protein 20. The target protein 20a, the cleaved peptide tag 20b, the protease 22 fused with the peptide tag and contaminant proteins 21E and 21F remained in the separation step are contained in the solution after reaction. A peptide tag 20b with a low isoelectric point is cleaved, thus the isoelectric point of the target protein 20a becomes high as compared to that of the fusion protein 20. The contaminant proteins 21E and 21F have an isoelectric point relatively close to that of the fusion protein 20, thus the target protein 20a has an isoelectric point higher than those of those proteins. Furthermore, the target protein 20a has an isoelectric point higher than those of the cleaved peptide tag 20b and the protease 22 fused with the peptide tag 22b. Therefore, when this solution is diluted to lower the salt concentration, and passed through the anion exchange resin 10, the cleaved peptide tag 20b, the protease fused with the peptide tag 22 and contaminant proteins 21E and 21F bind to the anion exchange resin 10, but the target protein 20a passes through without binding thereto. High purity target protein 20a can be acquired by collecting this flow-through fraction.

### EXAMPLES

Hereinbelow, the present invention will be further described in detail with reference to examples, but the present invention is not limited to these examples.

### Example 1 [Expression and Purification of Fusion Protein DE12-HRV3Csite-NQO1 (Construct 1; pI = 5.84)]

Peptide tag 1 (DE12) was fused to the N-terminal side of NAD(P)H dehydrogenase, quinone 1 human (NQO1; pI = 8.91) that is a target protein, and expression and purification of a fusion protein containing a cleavable site HRV3Csite recognized by protease HRV3C between NQO1 and DE12 were performed.

### (1) Construction of pM01_DE12 Vector

A pM01_DE12 vector in which the peptide tag DE12 was inserted into a pM01 vector was constructed.

Polynucleotides encoding DE12 (SEQ ID NOs.: 25 and 26) were annealed in pairs to prepare an insert part. A NheI site of the pM01 vector (SYSMEX CORPORATION) was treated with NheI (TAKARA BIO INC.), and the insert part was inserted using In-Fusion HD Cloning Kit (Clontech Laboratories, Inc.). Stellar Competent Cells were transformed with the constructed vector, then the obtained transformant was seeded on a LBA plate, and cultured at 37°C for 16 hrs. An ampicillin resistant transformant was selected from the obtained transformants according to normal procedure, and purification of plasmid was performed. In order to confirm a base sequence of the selected clone, the PCR reaction was performed by Big Dye Terminator v3.1 (Thermo Fisher Scientific Inc.), using primer 1 (SEQ ID NO.: 27) and primer 2 (SEQ ID NO.: 28) for sequence confirmation, then the base sequence was analyzed by a DNA sequencer (Thermo Fisher Scientific Inc.). A vector into which the insert part was introduced, that had no variation in other part, was defined as pM01_DE12.

### (2) Construction of pM01_DE12_HRV3Csite_NQO1

An expression vector pM01_DE12_HRV3Csite_NQ01 into which a polynucleotide (SEQ ID NO.: 29) encoding NQO1 (SEQ ID NO.: 9) was inserted into pM01_DE12 was constructed.

The polynucleotide encoding NQO1 and primers 3F (SEQ ID NO.: 30) and 3R (SEQ ID NO.: 31) corresponding to the insert part were prepared, and the insert part was amplified using KOD-Plus- (TOYOBO CO., LTD.) as a PCR enzyme, under the following PCR conditions. The amplified PCR product was migrated by 1.0% (w/v) agarose electrophoresis, and the part matched to the length of the insert part was cut out from the gel and purified to prepare an insert part. A SmaI site of the pM01_DE12 vector was treated with SmaI (TAKARA BIO INC.), and the insert part was inserted using In-Fusion HD Cloning Kit (Clontech Laboratories, Inc.). Stellar Competent Cells (Clontech Laboratories, Inc.) were transformed with the constructed vector, then the obtained transformant was seeded on a LBA plate. The transformant was cultured at 37°C for 16 hrs. An ampicillin resistant transformant was selected from the obtained transformants according to normal procedure, and purification of plasmid was performed. In order to confirm a base sequence of the selected clone, the PCR reaction was performed by Big Dye Terminator v3.1 (Thermo Fisher Scientific Inc.), using primers 1 and 2 for sequence confirmation, then the base sequence was analyzed by a DNA sequencer (Thermo Fisher Scientific Inc.). A vector into which the insert part was introduced, that had no variation in other part, was defined as pM01_DE12_HRV3Csite_NQO1.

### <PCR Conditions>

Reaction 1: 94.0°C, 2-minute reaction 2: 94.0°C, 15-second reaction 3: 52.0°C, 30-second reaction 4: 68.0°C, 1-minute reaction 5: reaction 2 to reaction 4 are repeated 30 times per 1 kbp

### (3) Preparation of Recombinant Baculovirus and Expression of Fusion Protein DE12-HRV3Csite-NQO1

pM01_DE12_HRV3Csite_NQO1 and baculovirus DNA were introduced into silkworm culture cells (BmN cells), and homologous recombination of virus was performed. The culture cells were cultured for 6 days, and the recombinant virus was collected. Thereafter, a virus solution diluted 50 times with MilliQ water was inoculated into pupa of silkworm, and the pupa was incubated for 6 days.

### (4) Purification of DE12-HRV3Csite-NQO1

After incubation, the pupa of silkworm was collected, and 50 mL of a buffer (20 mM Tris-HCl (pH 8.0), 150 mM NaCl, 1 tablet Complete EDTA free (Roche), phenylthiourea) was added per a pupa to homogenate the pupa. The solution was centrifuged (8,000 g, 10 minutes), then the supernatant fraction was filtered with 0.8 uM of a filter agent, and the filtrate was collected. The collected filtrate was loaded on an ion exchange resin (Hitrap Q HP (1 mL)), and purified using a mixed solution of 20 mM Tris-HCl (pH 8.0, 150 mM NaCl; buffer A) and 20 mM Tris-HCl (pH 8.0, 1000 mM NaCl; buffer B). First, only buffer A (NaCl concentration of 150 mM) was flown through the ion exchange resin (EL0), and a fraction eluted with a 3:1 mixed solution of buffer A:buffer B (NaCl concentration of 363 mM) (EL1), a fraction eluted with a 1:1 mixed solution of buffer A:buffer B (NaCl concentration of 575 mM) (EL2), a fraction eluted with a 1:3 mixed solution of buffer A:buffer B (NaCl concentration of 788 mM) (EL3), and finally, a fraction eluted with only buffer B (NaCl concentration of 1000 mM) (EL4) were each collected.

### (5) Detection of DE12-HRV3Csite-NQO1

SDS (Sodium dodecyl sulfate)-polyacrylamide gel electrophoresis (SDS-PAGE) was performed using 5 to 20% gradient gel and an electrophoresis apparatus (APRO Science Inc.). A sample and β-ME Sample Treatment for Tris SDS (COSMO BIO Co., Ltd. were mixed in a volume ratio of 1:1, and treated at 100°C for 3 minutes, then 5 uL of the resulting solution was loaded on the gel. As a molecular weight marker, 2.5 uL of Blue Star (NIPPON Genetics Co., Ltd.) was loaded on the gel. The gel loaded with the sample was migrated at a voltage of 400 V for 14 minutes, and then transferred to a membrane using Trans-Blot Turbo Transfer System (Bio-Rad Laboratories, Inc.). The transferred membrane was set in i-Bind system (Thermo Fisher Scientific Inc.), and an antigen-antibody reaction was performed. In the antigen-antibody reaction, an ANTI-FLAG (registered trademark) M2-Peroxidase (HRP) antibody (Merck & Co., Inc.) or an Anti-NQO1 antibody (Cell Signaling Technology, Inc.) and Anti-IgG (H+L chain) (Mouse) pAb-HRP (Beckman Coulter, Inc.) were diluted 2000 times and used. Also, Luminata Forte (Merck & Co., Inc.) as a HRP reagent for detection and Gel Doc XR+ system (Bio-Rad Laboratories, Inc.) as a detector were used.

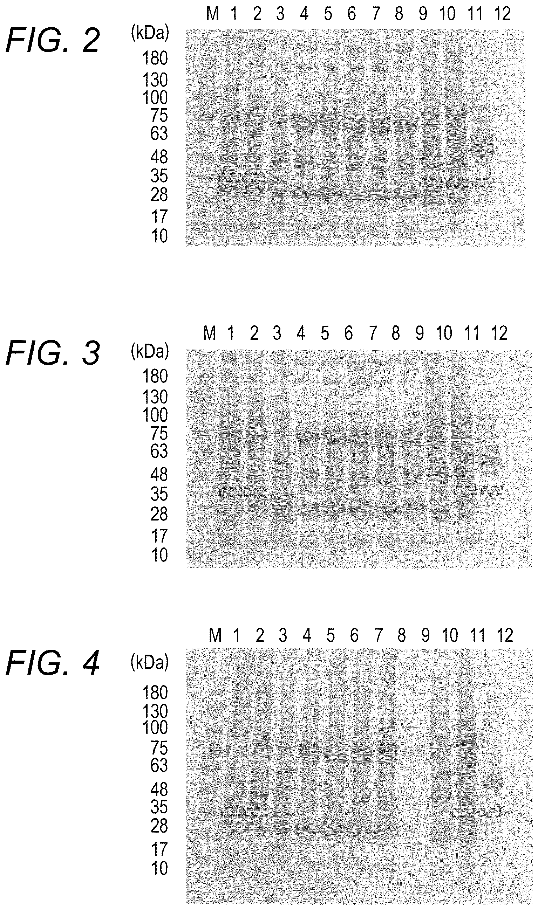

After performing western blotting, the used membrane was stained by GelCode Blue Stain Reagent (Thermo Fisher Scientific Inc.), and excess staining was bleached, then the image was photographed using Gel Doc XR+ system (Bio-Rad Laboratories, Inc.). The results were shown in Fig. 2. In Fig. 2, the results of electrophoresis are shown as follows; M: molecular weight marker, lane 1: homogenate solution, lane 2: supernatant fraction after centrifugation, lane 3: precipitated fraction after centrifugation, lanes 4 to 7: flow-through fractions 1 to 4 of the ion exchange resin, lane 8: elution fraction by buffer A (NaCl concentration of 150 mM) (EL0), lane 9: elution fraction by a 3:1 mixed solution of buffer A:buffer B (NaCl concentration of 363 mM) (EL1), lane 10: elution fraction by a 1:1 mixed solution of buffer A:buffer B (NaCl concentration of 575 mM) (EL2), lane 11: elution fraction by a 1:3 mixed solution of buffer A:buffer B (NaCl concentration of 788 mM) (EL3), and lane 12: elution fraction by buffer B (NaCl concentration of 1000 mM) (EL4). The part surrounded by a broken line shows a band containing the target protein. Explanation of each lane is common in Figs. 3 to 15.

### Example 2 [Expression and Purification of Fusion Protein DE18-HRV3Csite-NQO1 (Construct 2; pI = 5.03)]

Peptide tag 2 (DE18) was fused to the N-terminal side of NQO1 that is a target protein, and expression and purification of a fusion protein containing HRV3Csite between NQO1 and DE18 were performed.

The same procedures were carried out as in Example 1, except for preparing an insert part using polynucleotides (SEQ ID NOs.: 32 and 33) encoding DE18 (SEQ ID NO.: 2) in place of the polynucleotides encoding the peptide tag DE12, to construct a pM01_DE18 vector.

Further, pM01_DE18_HRV3Csite_NQO1 was constructed, and a recombinant baculovirus was prepared in the same manner as in Example 1, then expression, purification and detection of fusion protein DE18-HRV3Csite-NQO1 were performed. The results were shown in Fig. 3.

### Example 3 [Expression and Purification of Fusion Protein DE24-HRV3Csite-NQO1 (Construct 3; pI = 4.78)]

Peptide tag 3 (DE24) was fused to the N-terminal side of NQO1 that is a target protein, and expression and purification of a fusion protein containing HRV3Csite between NQO1 and DE24 were performed.
(1) Construction of pM01_DE24 Vector
   A pM01_DE24 vector in which a polynucleotide encoding peptide tag DE24 was inserted into a pM01 vector was constructed as below.
   The polynucleotide (SEQ ID NO.: 34) encoding peptide tag 6 (DED; SEQ ID NO.: 6) and primers 4F (SEQ ID NO.: 35) and 4R (SEQ ID NO.: 36) corresponding to the insert part were prepared, and the insert part was amplified using KOD-Plus- (TOYOBO CO., LTD.) as a PCR enzyme, under the same PCR conditions as in Example 1. The amplified PCR product was migrated by 1.0% (w/v) agarose electrophoresis, and the part matched to the length of the insert part was cut out from the gel and purified to prepare an insert part. A SmaI site of the pM01 vector (SYSMEX CORPORATION) was treated with SmaI (TAKARA BIO INC.), and the insert part was inserted using In-Fusion HD Cloning Kit (Clontech Laboratories, Inc.). Stellar Competent Cells (Clontech Laboratories, Inc.) were transformed with the constructed vector, then the obtained transformant was seeded on a LBA plate. The transformant was cultured at 37°C for 16 hrs. An ampicillin resistant transformant was selected from the obtained transformants according to normal procedure, and purification of plasmid was performed. In order to confirm a base sequence of the selected clone, the PCR reaction was performed by Big Dye Terminator v3.1 (Thermo Fisher Scientific Inc.), using primers 1 and 2 for sequence confirmation, then the base sequence was analyzed by a DNA sequencer (Thermo Fisher Scientific Inc.). A vector into which the insert part was introduced, that had no variation in other part, was defined as pM01_DE24.
(2) pM01_DE24_HRV3Csite_NQO1 was constructed, and a recombinant baculovirus was prepared in the same manner as in Example 1, then expression, purification and detection of fusion protein DE24-HRV3Csite-NQO1 were performed. The results were shown in Fig. 4.

### Example 4 [Expression and Purification of Fusion Protein DE30-HRV3Csite-NQO1 (Construct 4; pI = 4.61)]

Peptide tag 4 (DE30) was fused to the N-terminal side of NQO1 that is a target protein, and expression and purification of a fusion protein containing HRV3Csite between NQO1 and DE30 were performed.

The same procedures were carried out as in Example 1, except for preparing an insert part using polynucleotides (SEQ ID NOs.: 37 and 38) encoding DE30 (SEQ ID NO.: 4) in place of the polynucleotides encoding the peptide tag DE12, to construct a pM01_DE30 vector.

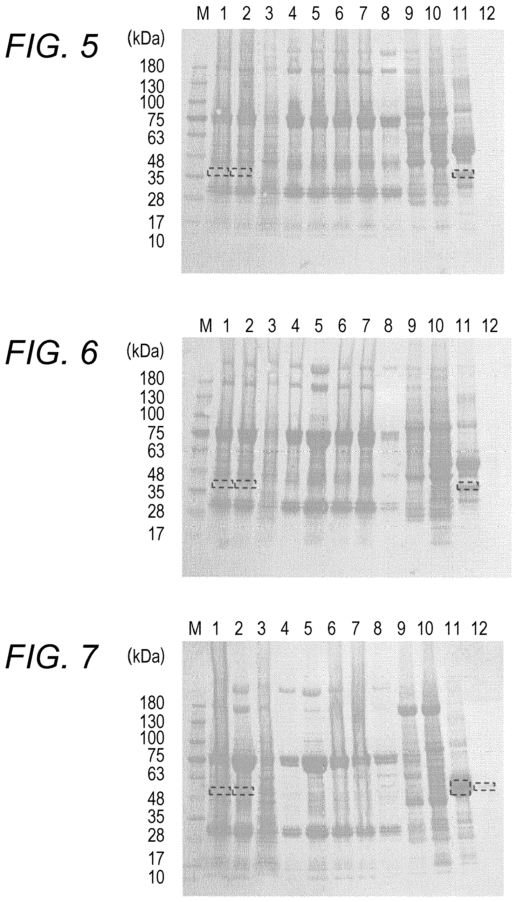

Further, pM01_DE30_HRV3Csite_NQO1 was constructed, and a recombinant baculovirus was prepared in the same manner as in Example 1, then expression, purification and detection of fusion protein DE30-HRV3Csite-NQO1 were performed. The results were shown in Fig. 5.

### Example 5 [Expression and Purification of Fusion Protein DE36-HRV3Csite-NQO1 (Construct 5; pI = 4.49)]

Peptide tag 5 (DE36) was fused to the N-terminal side of NQO1 that is a target protein, and expression and purification of a fusion protein containing HRV3Csite between NQO1 and DE36 were performed.

The same procedures were carried out as in Example 1, except for preparing an insert part using polynucleotides (SEQ ID NOs.: 39 and 40) encoding DE36 (SEQ ID NO.: 5) in place of the polynucleotides encoding the peptide tag DE12, to construct a pM01_DE36 vector.

Further, pM01_DE36_HRV3Csite_NQO1 was constructed, and a recombinant baculovirus was prepared in the same manner as in Example 1, then expression, purification and detection of fusion protein DE36-HRV3Csite-NQO1 were performed. The results were shown in Fig. 6.

### Example 6 [Expression and Purification of Fusion Protein DED-HRV3Csite-NQO1 (Construct 6; pI = 4.26)]

Peptide tag 6 (DED) was fused to the N-terminal side of NQO1 that is a target protein, and expression and purification of a fusion protein containing HRV3Csite between NQO1 and DED were performed.
(1) Construction of pHS01_DED Vector
   A pHS01_DED vector in a peptide tag DED (SEQ ID NO.: 6) was inserted into a pHS01 vector was constructed as below.
   The polynucleotide (SEQ ID NO.: 34) encoding the peptide tag DED and primers 5F (SEQ ID NO.: 41) and 5R (SEQ ID NO.: 42) corresponding to the insert part were prepared, and the insert part was amplified using KOD-Plus- (TOYOBO CO., LTD.) as a PCR enzyme, under the same PCR conditions as in Example 1. The amplified PCR product was migrated by 1.0% (w/v) agarose electrophoresis, and the part matched to the length of the insert part was cut out from the gel and purified to prepare an insert part. A NheI site of the pHS01 vector (SYSMEX CORPORATION) was treated with NheI (TAKARA BIO INC.), and the insert part was inserted using In-Fusion HD Cloning Kit (Clontech Laboratories, Inc.). Stellar Competent Cells were transformed with the constructed vector, then the obtained transformant was seeded on a LBA plate. The transformant was cultured at 37°C for 16 hrs. An ampicillin resistant transformant was selected from the obtained transformants according to normal procedure, and purification of plasmid was performed. In order to confirm a base sequence of the selected clone, the PCR reaction was performed by Big Dye Terminator v3.1 (Thermo Fisher Scientific Inc.), using primers 1 and 2 for sequence confirmation, then the base sequence was analyzed by a DNA sequencer (Thermo Fisher Scientific Inc.). A vector into which the insert part was introduced, that had no variation in other part, was defined as pHS01_DED.
(2) HS01_DED_HRV3Csite_NQO1 was constructed, and a recombinant baculovirus was prepared in the same manner as in Example 1, then expression, purification and detection of fusion protein DED-HRV3Csite-NQO1 were performed. The results were shown in Fig. 7.

### Example 7 [Expression and Purification of Fusion Protein DES-HRV3Csite-NQO1 (Construct 7; pI = 4.55)]

Peptide tag 7 (DES) was fused to the N-terminal side of NQO1 that is a target protein, and expression and purification of a fusion protein containing HRV3Csite between NQO1 and DES were performed.

The same procedures were carried out as in Example 6, except for using primers 6F (SEQ ID NO.: 43) and 6R (SEQ ID NO.: 44) as the primers corresponding to the insert part, to construct pHS01_DES.

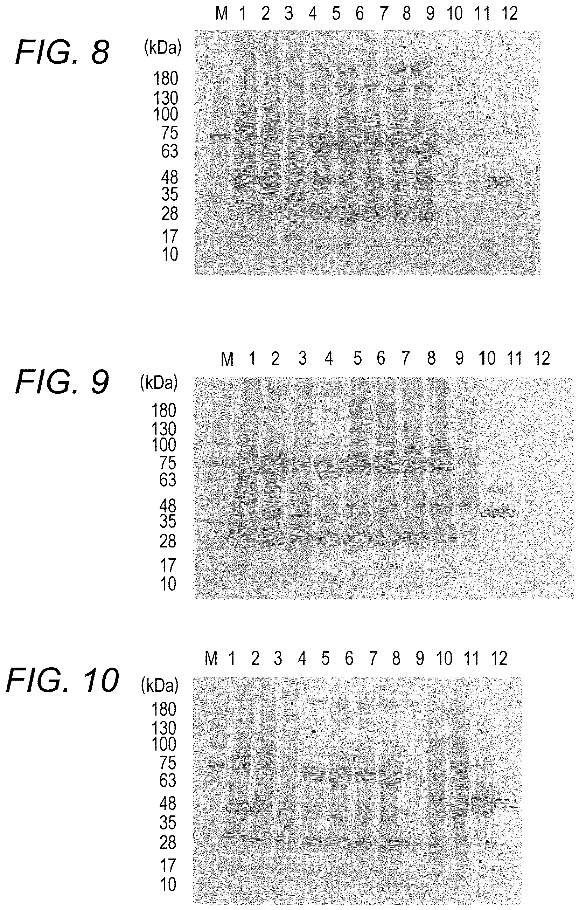

pHS01_DES_HRV3Csite_NQO1 was constructed, and a recombinant baculovirus was prepared in the same manner as in Example 1, then expression, purification and detection of fusion protein DES-HRV3Csite-NQO1 were performed. The results were shown in Fig. 8.

### Example 8 [Expression and Purification of Fusion Protein E024-HRV3Csite-NQO1 (Construct 8; pI = 4.73)]

Peptide tag 8 (EO24) was fused to the N-terminal side of NQO1 that is a target protein, and expression and purification of a fusion protein containing HRV3Csite between NQO1 and EO24 were performed.
(1) Construction of pHS01_EO24
   Polynucleotides encoding peptide tag EO24 (SEQ ID NOs.: 45 and 46) were annealed in pairs to prepare an insert part. A NheI site of the pHS01 vector (SYSMEX CORPORATION) was treated with NheI (TAKARA BIO INC.), and the insert part was inserted using In-Fusion HD Cloning Kit (Clontech Laboratories, Inc.). Stellar Competent Cells (Clontech Laboratories, Inc.) were transformed with the constructed vector, then the obtained transformant was seeded on a LBA plate. The transformant was cultured at 37°C for 16 hrs. An ampicillin resistant transformant was selected from the obtained transformants according to normal procedure, and purification of plasmid was performed. In order to confirm a base sequence of the selected clone, the PCR reaction was performed by Big Dye Terminator v3.1 (Thermo Fisher Scientific Inc.), using primers 1 and 2 for sequence confirmation, then the base sequence was analyzed by a DNA sequencer (Thermo Fisher Scientific Inc.). A vector into which the insert part was introduced, that had no variation in other part, was defined as pHS01_EO24.
(2) pHS01_EO24_HRV3Csite_NQO1 was constructed, and a recombinant baculovirus was prepared in the same manner as in Example 1, then expression, purification and detection of fusion protein EO24-HRV3Csite-NQO1 were performed. The results were shown in Fig. 9.

### Example 9 [Expression and Purification of Fusion Protein NQO1-HRV3Csite-DED (Construct 9; pI = 4.26)]

The peptide tag DED was fused to the C-terminal side of NQO1 that is a target protein, and expression and purification of a fusion protein containing HRV3Csite between NQO1 and DED were performed.
(1) Construction of pHS02_DED Vector
   pHS02_DE12 Vector in which a polynucleotide encoding the peptide tag DED was inserted into pHS02 vector was constructed as below.
   The polynucleotide (SEQ ID NO.: 34) encoding the peptide tag DED and primers 7F (SEQ ID NO.: 47) and 7R (SEQ ID NO.: 48) corresponding to the insert part were prepared, and the insert part was amplified using KOD-Plus- (TOYOBO CO., LTD.) as a PCR enzyme, under the same PCR conditions as in Example 1. The amplified PCR product was migrated by 1.0% (w/v) agarose electrophoresis, and the part matched to the length of the insert part was cut out from the gel and purified to prepare an insert part. A NheI site of the pHS02 vector (SYSMEX CORPORATION) was treated with NheI (TAKARA BIO INC.), and the insert part was inserted using In-Fusion HD Cloning Kit (Clontech Laboratories, Inc.). Stellar Competent Cells (Clontech Laboratories, Inc.) were transformed with the constructed vector, then the obtained transformant was seeded on a LBA plate. The transformant was cultured at 37°C for 16 hrs. An ampicillin resistant transformant was selected from the obtained transformants according to normal procedure, and purification of plasmid was performed. In order to confirm a base sequence of the selected clone, the PCR reaction was performed by Big Dye Terminator v3.1 (Thermo Fisher Scientific Inc.), using primers 1 and 2 for sequence confirmation, then the base sequence was analyzed by a DNA sequencer (Thermo Fisher Scientific Inc.). A vector into which the insert part was introduced, that had no variation in other part, was defined as pHS02_DED.
(2) The same procedures were carried out as in Example 1, except for using primers 8F (SEQ ID NO.: 49) and 8R (SEQ ID NO.: 50) as the primers corresponding to the insert part, to construct pHS02_NQO1_HRV3Csite_DED.
(3) Further, a recombinant baculovirus was prepared in the same manner as in Example 1, and expression, purification and detection of fusion protein NQO1-HRV3Csite-DED were performed. The results were shown in Fig. 10.

### Example 10 [Expression and Purification of Fusion Protein NQO1-HRV3Csite-DES (Construct 10; pI = 4.55)]

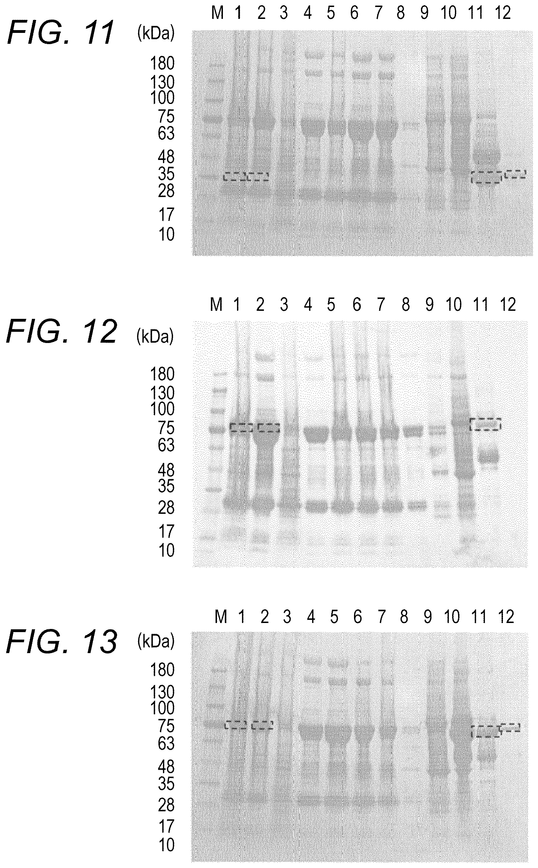

Peptide tag DES was fused to the C-terminal side of NQO1 that is a target protein, and expression and purification of a fusion protein containing HRV3Csite between NQO1 and DES were performed.
(1) The same procedures were carried out as in Example 9, except for using 9F (SEQ ID NO.: 51) and 9R (SEQ ID NO.: 52) as the primers, to construct pHSO2_DES.
(2) The same procedures were carried out as in Example 9 to construct pHS01 NQO1_HRV3Csite_DES.
(3) A recombinant baculovirus was prepared in the same manner as in Example 1, and expression, purification and detection of fusion protein NQO1-HRV3Csite-DES were performed. The results were shown in Fig. 11.

### Example 11 [Expression and Purification of Fusion Protein DED-HRV3Csite-Luciferase (Construct 11; pI = 4.47)]

(1) Construction of pHS01_DED Vector
   The same procedures were carried out as in Example 6 to construct pHS01_DED.
(2) Construction of pHS01_DED_HRV3Csite_Luciferase
   The polynucleotide (SEQ ID NO.: 53) encoding Luciferase and primers 10F (SEQ ID NO.: 54) and 10R (SEQ ID NO.: 55) corresponding to the insert part were prepared, and the insert part was amplified using KOD-Plus- (TOYOBO CO., LTD.) as a PCR enzyme, under the same PCR conditions as in Example 1. The amplified PCR product was migrated by 1.0% (w/v) agarose electrophoresis, and the part matched to the length of the insert part was cut out from the gel and purified to prepare an insert part. A SmaI site of the pHS01_DED vector was treated with SmaI (TAKARA BIO INC.), and the insert part was inserted using In-Fusion HD Cloning Kit (Clontech Laboratories, Inc.). Stellar Competent Cells (Clontech Laboratories, Inc.) were transformed with the constructed vector, then the obtained transformant was seeded on a LBA plate. The transformant was cultured at 37°C for 16 hrs. An ampicillin resistant transformant was selected from the obtained transformants according to normal procedure, and purification of plasmid was performed. In order to confirm a base sequence of the selected clone, the PCR reaction was performed by Big Dye Terminator v3.1 (Thermo Fisher Scientific Inc.), using primers 1 and 2 for sequence confirmation, then the base sequence was analyzed by a DNA sequencer (Thermo Fisher Scientific Inc.). A vector into which the insert part was introduced, that had no variation in other part, was defined as pHS01_DED_HRV3Csite_Luciferase.
(3) A recombinant baculovirus was prepared in the same manner as in Example 1, and expression, purification and detection of fusion protein DED-HRV3Csite-Luciferase were performed. The results were shown in Fig. 12.

### Example 12 [Expression and Purification of Fusion Protein DES-HRV3Csite-Luciferase (Construct 12; pI = 4.75)]

The same procedures were carried out as in Example 11, except for using a pHS01_DES vector constructed in Example 7 in place of the pHS01_DED vector, to construct pHS02_DES_HRV3Csite_Luciferase.

A recombinant baculovirus was prepared in the same manner as in Example 1, and expression, purification and detection of fusion protein DES-HRV3Csite-Luciferase were performed. The results were shown in Fig. 13.

### Example 13 [Expression and Purification of Fusion Protein DED-HRV3C (Construct 13; pI = 4.75)]

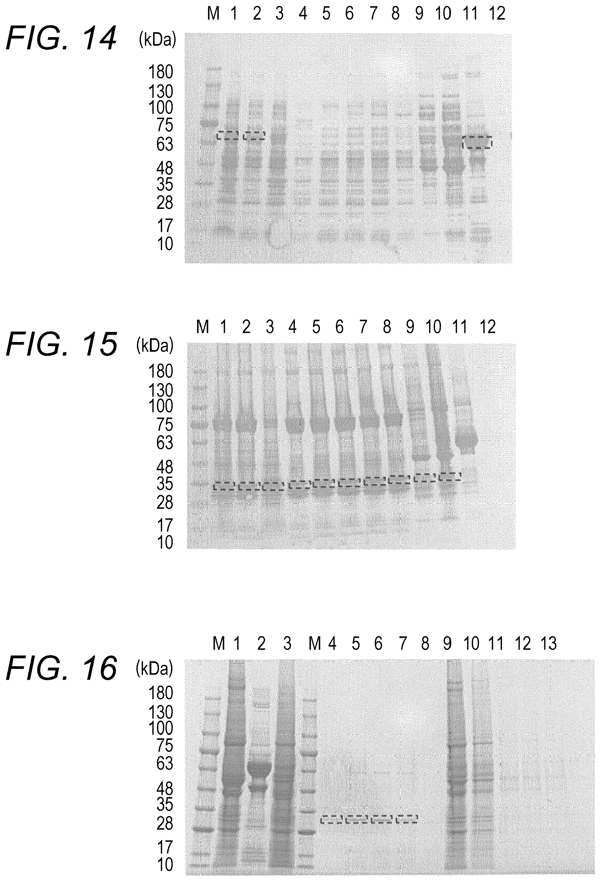

(1) Construction of pET17b_DED_HRV3C
   The polynucleotide (SEQ ID NO.: 34) encoding the peptide tag DED, a polynucleotide (SEQ ID NO.: 56) encoding HRV3C, primers 11F and 11R (SEQ ID NOs.: 57 and 58; DED) corresponding to the insert part and primers 12F and 12R (SEQ ID NOs.: 59 and 60; HRV3C) were prepared, and the insert part was amplified using KOD-Plus- (TOYOBO CO., LTD.) as a PCR enzyme, under the same PCR conditions as in Example 1. The amplified PCR product was migrated by 1.0% (w/v) agarose electrophoresis, and the part matched to the length of the insert part was cut out from the gel and purified to prepare an insert part. A SmaI site and a NheI site of the pHS01 vector (SYSMEX CORPORATION) were treated with SmaI (TAKARA BIO INC.) and NheI (TAKARA BIO INC.), and the two insert parts were inserted using In-Fusion HD Cloning Kit (Clontech Laboratories, Inc.). Stellar Competent Cells (Clontech Laboratories, Inc.) were transformed with the constructed vector, then the obtained transformant was seeded on a LBA plate. The transformant was cultured at 37°C for 16 hrs. An ampicillin resistant transformant was selected from the obtained transformants according to normal procedure, and purification of plasmid was performed. In order to confirm a base sequence of the selected clone, the PCR reaction was performed by Big Dye Terminator v3.1 (Thermo Fisher Scientific Inc.), using primers 1 and 2 for sequence confirmation, then the base sequence was analyzed by a DNA sequencer (Thermo Fisher Scientific Inc.). A vector into which the insert part was introduced, that had no variation in other part, was defined as pHS01_DED_HRV3C.
   Next, an insert for pET17b was prepared. Primers 13F (SEQ ID NO.: 61) and 13R (SEQ ID NO.: 62) were prepared, and the primers were amplified using pHS01_DED_HRV3C as a template, and using KOD-Plus- (TOYOBO CO., LTD.) as a PCR enzyme, under the same PCR conditions as in Example 1. The amplified PCR product was migrated by 1.0% (w/v) agarose electrophoresis, and the part matched to the length of the insert part was cut out from the gel and purified to prepare an insert part. A NdeI site of the pET17b Vector (Novagen, Inc.) was treated with NdeI (TAKARA BIO INC.), and the insert part was inserted using In-Fusion HD Cloning Kit (Clontech Laboratories, Inc.). Stellar Competent Cells (Clontech Laboratories, Inc.) were transformed with the constructed vector, then the obtained transformant was seeded on a LBA plate. The transformant was cultured at 37°C for 16 hrs. An ampicillin resistant transformant was selected from the obtained transformants according to normal procedure, and purification of plasmid was performed. In order to confirm a base sequence of the selected clone, the PCR reaction was performed by Big Dye Terminator v3.1 (Thermo Fisher Scientific Inc.), using primers 14 (SEQ ID NO.: 63) and 15 (SEQ ID NO.: 64) for sequence confirmation, then the base sequence was analyzed by a DNA sequencer (Thermo Fisher Scientific Inc.). A vector into which the insert part was introduced, that had no variation in other part, was defined as pET17b_DED_HRV3C.
(2) Expression and Purification of DED-HRV3C
   E. coli was transformed using pET17b_DED_HRV3C, and 1.5 L of an LB medium containing ampicillin was cultured at 37°C and cooled to 15°C around a turbidity OD of about 0.6, then IPTG was added thereto to induce expression of protein. After 16 hours, bacteria were collected by centrifugation (8,000 g, 15 minutes) and frozen at -80°C. 120 mL of a buffer (20 mM Tris-HCl (pH 8.0), 150 mM NaCl, 1 tablet Complete EDTA free (Roche)) was added to the frozen E. coli, and E. coli was crushed by ultrasonic wave. The crushed solution was centrifuged (15,000 g x 30 minutes), then the supernatant fraction was filtered with 0.8 uM of a filter agent, and the filtrate was collected. The filtrate was loaded on an ion exchange resin (Hitrap Q HP (1 mL)), and purified using a mixed solution of 20 mM Tris-HCl (pH 8.0, 150 mM NaCl; buffer A) and 20 mM Tris-HCl (pH 8.0, 1000 mM NaCl; buffer B). First, only buffer A (NaCl concentration of 150 mM) was flown through the ion exchange resin (EL0), and a fraction eluted with a 3 : 1 mixed solution of buffer A:buffer B (NaCl concentration of 363 mM) (EL1), a fraction eluted with a 1 : 1 mixed solution of buffer A : buffer B (NaCl concentration of 575 mM) (EL2), a fraction eluted with a 1 : 3 mixed solution of buffer A : buffer B (NaCl concentration of 788 mM) (EL3), and finally, a fraction eluted with only buffer B (NaCl concentration of 1000 mM) (EL4) were each collected.
(3) The same procedures were carried out as in Example 1 to detect a fusion protein. The results were shown in Fig. 14. In Fig. 14, the results of electrophoresis are shown as follows; M: molecular weight marker, lane 1: homogenate solution, lane 2: supernatant fraction after centrifugation, lane 3: precipitated fraction after centrifugation, lanes 4 to 7: flow-through fractions 1 to 4 of the ion exchange resin, lane 8: elution fraction by buffer A (NaCl concentration of 150 mM) (EL0), lane 9: elution fraction by a 3 : 1 mixed solution of buffer A : buffer B (NaCl concentration of 363 mM) (EL1), lane 10: elution fraction by a 1 : 1 mixed solution of buffer A : buffer B (NaCl concentration of 575 mM) (EL2), lane 11: elution fraction by a 1 : 3 mixed solution of buffer A : buffer B (NaCl concentration of 788 mM) (EL3), and lane 12: elution fraction by buffer B (NaCl concentration of 1000 mM) (EL4).

### Comparative Example 1 [Expression and Purification of Fusion Protein DE6-HRV3Csite-NQO1 (pI = 6.4)]

Peptide tag DE6 was fused to the N-terminal side of NQO1 that is a target protein, and expression and purification of a fusion protein containing HRV3Csite between NQO1 and DE6 were performed.

The same procedures were carried out as in Example 1, except for preparing an insert part using polynucleotides (SEQ ID NOs.: 66 and 67) encoding DE6 (EEEDDD; SEQ ID NO.: 65) in place of the polynucleotides encoding the peptide tag DE12, to construct a pM01_DE6 vector.

Further, pM01_DE6_HRV3Csite_NQO1 was constructed, and a recombinant baculovirus was prepared in the same manner as in Example 1, then expression, purification and detection of fusion protein DE6-HRV3Csite-NQO1 were performed. The results were shown in Fig. 15.

### (Regarding Results of Examples 1 to 13 and Comparative Example 1)

In Comparative Example 1 (DE6-HRV3Csite-NQO1, pI = 6.4), a fusion protein was contained in every fraction, and separation was insufficient. On the other hand, Examples 1 to 13 all showed good separation. Particularly, a fusion protein binding a peptide tag with 18 or more acidic amino acid residues was held in the ion exchange resin up to a high salt concentration of 500 mM or more, and separation from contaminant proteins was improved (Examples 2 to 13). Also in all three target proteins, good separation was obtained (Examples 6 to 7 and 11 to 13). The peptide tag, even the peptide tag containing both aspartic acid residue and glutamic acid residue as acidic amino acid residues, or the peptide tag only containing either one, equally showed good separation (Examples 3 and 8). Even when the binding position of the peptide tag is either N-terminal side or C-terminal side of the target protein, separation was good (Examples 6 to 7 and 9 to 10).

### Example 14 [Separation and Detection of Target Protein NQ1 from Fusion Protein DE12-HRV3Csite-NQO1]

Protease DED-HRV3C was reacted with DE12-HRV3Csite-NQO1 to cleave DE12 from DE12-HRV3Csite-NQO1.

To Fraction EL2 collected in Example 1 was mixed a 1/10 volume of Fraction EL3 collected in Example 13, and the mixture was allowed to stand at 4°C. After 16 hours, 20 mM Tris-HCl (pH 8.0) was added to this reaction solution to be diluted to a NaCl concentration of about 250 mM, and the diluted solution was loaded on an ion exchange resin (Hitrap Q HP (1 mL)) equilibrated with 20 mM Tris-HCl (pH 8.0), 150 mM NaCl. Flow-through fractions were collected (Fr. 1 to 6 (NaCl concentration of about 250 mM)), and fractions finally eluted (Fr. 7 to 10) with only buffer B (NaCl concentration of about 1000 mM) were each collected.

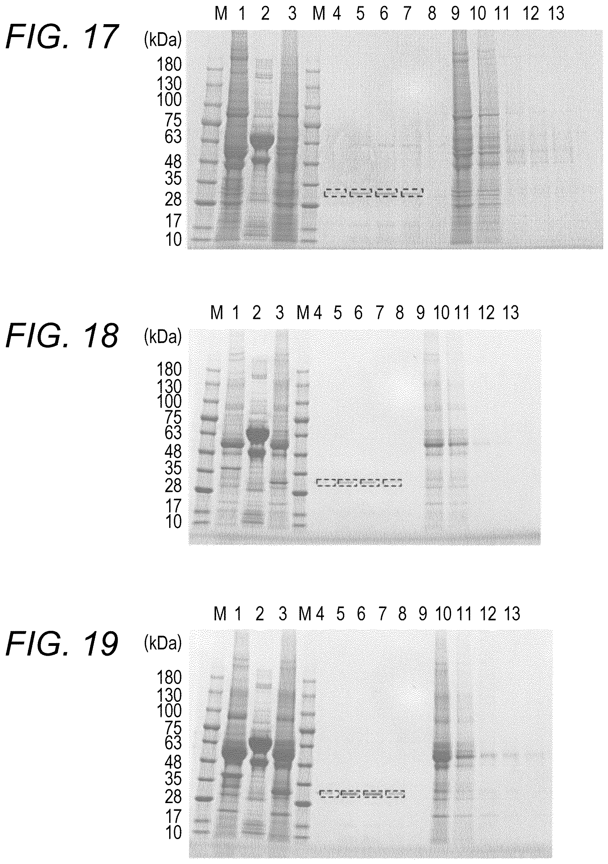

SDS-PAGE was performed using 5 to 20% gradient gel and an electrophoresis apparatus (APRO Science Inc.). Each collected fraction and β-ME Sample Treatment for Tris SDS (COSMO BIO Co., Ltd.) were mixed in a volume ratio of 1:1, and treated at 100°C for 3 minutes, then 5 uL of the resulting solution was loaded on the gel. As a molecular weight marker, 2.5 uL of Blue Star (NIPPON Genetics Co., Ltd.) was loaded on the gel. The gel loaded with the sample was migrated at a voltage of 400 V for 14 minutes, and then the gel was stained by GelCode Blue Stain Reagent (Thermo Fisher Scientific Inc.), and excess staining was bleached, then the image was photographed using Gel Doc XR+ system (Bio-Rad Laboratories, Inc.). The results were shown in Fig. 16. In Fig. 16, the results of electrophoresis are shown as follows; M: molecular weight marker, lane 1: EL2 (Examples 1 and 8) or EL3 (Examples 2 to 7 and 11 to 12), lane 2: EL3 of Example 13, lane 3: a mixed reaction liquid of EL2 or EL3 of Examples 1 to 12, and EL3 of Example 13, lanes 4 to 8: flow-through fractions 1 to 5 of the ion exchange resin, and lanes 9 to 13: elution fractions 1 to 5 by buffer B. Explanation of each lane is common in Figs. 17 to 25.

### Example 15

The same procedures were carried out as in Example 14, except for using EL2 of Example 2 in place of EL2 of Example 1, to detect a target protein NQO1. The results were shown in Fig. 17.

### Example 16

The same procedures were carried out as in Example 14, except for using EL3 of Example 3 in place of EL2 of Example 1, to detect a target protein NQO1. The results were shown in Fig. 18.

### Example 17

The same procedures were carried out as in Example 14, except for using EL3 of Example 4 in place of EL2 of Example 1, to detect a target protein NQO1. The results were shown in Fig. 19.

### Example 18

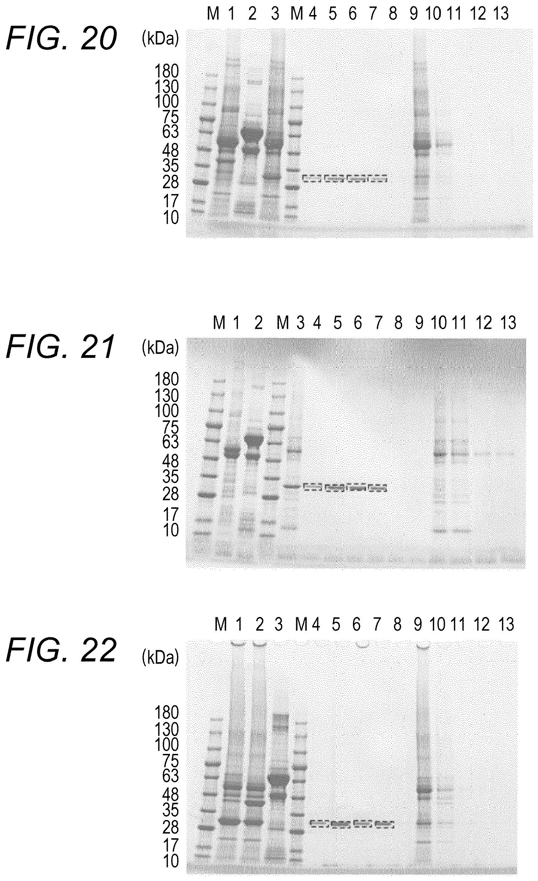

The same procedures were carried out as in Example 14, except for using EL3 of Example 5 in place of EL2 of Example 1, to detect a target protein NQO1. The results were shown in Fig. 20.

### Example 19

The same procedures were carried out as in Example 14, except for using EL3 of Example 6 in place of EL2 of Example 1, to detect a target protein NQO1. The results were shown in Fig. 21.

### Example 20

The same procedures were carried out as in Example 14, except for using EL3 of Example 7 in place of EL2 of Example 1, to detect a target protein NQO1. The results were shown in Fig. 22.

### Example 21

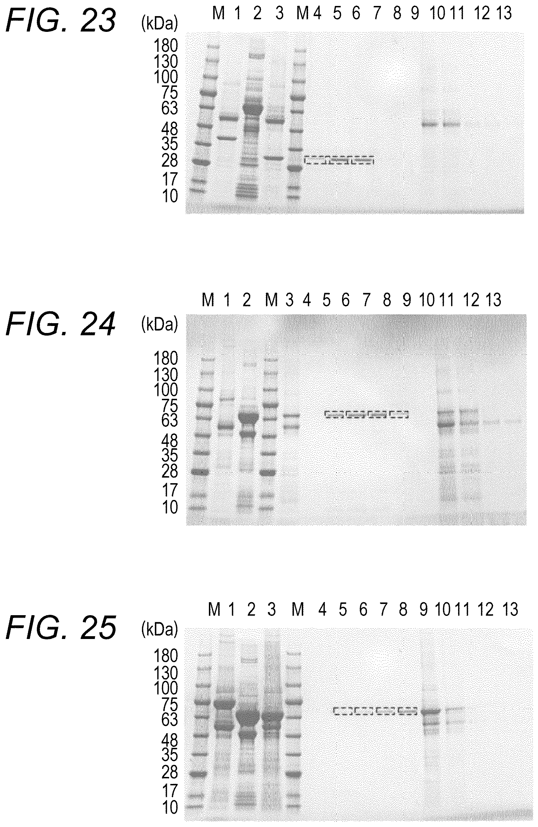

The same procedures were carried out as in Example 14, except for using EL2 of Example 8 in place of EL2 of Example 1, to detect a target protein NQO1. The results were shown in Fig. 23.

### Example 22

The same procedures were carried out as in Example 14, except for using EL3 of Example 11 in place of EL2 of Example 1, to detect a target protein Luciferase. The results were shown in Fig. 24.

### Example 23

The same procedures were carried out as in Example 14, except for using EL3 of Example 12 in place of EL2 of Example 1, to detect a target protein Luciferase. The results were shown in Fig. 25.

### (Regarding Results of Examples 14 to 23)

Based on the results of Examples 14 to 23, it was shown that the protease is reacted with the fusion protein, whereby a target protein NQO1 or Luciferase can be acquired in the flow-through fraction of the anion exchange resin.

### Example 24 [Evaluation of Effect on Separation due to pH of Buffer]

The same procedures were carried out as in Example 6 to construct pHS01_DED_HRV3Csite_NQO1.

pHS01_DED_HRV3Csite_NQO1 and baculovirus DNA were introduced into silkworm culture cells (BmN cells), and homologous recombination of virus was performed. The culture cells were cultured for 6 days, and the recombinant virus was collected. Thereafter, a virus solution diluted 50 times with MilliQ water was inoculated into pupa of silkworm, and the pupa was incubated for 6 days. The pupa of silkworm was collected, and 50 mL of a buffer (20 mM PIPES (pH 6.1), 150 mM NaCl, 1 tablet Complete EDTA free (Roche), phenylthiourea) was added per a pupa to homogenate the pupa. The solution was centrifuged (8,000 g, 10 minutes), then the supernatant fraction was filtered with 0.8 uM of a filter agent, and the filtrate was collected. The filtrate was loaded on an ion exchange resin (Hitrap Q HP (1 mL)), and purified using a mixed solution of 20 mM PIPES (pH 6.1, 150 mM NaCl; buffer C) and 20 mM PIPES (pH 6.1, 1000 mM NaCl; buffer D). First, only buffer C (NaCl concentration of 150 mM) was flown through the ion exchange resin (EL0), and a fraction eluted with a solution with a mixing ratio of buffer C : buffer D of 3 : 1 (NaCl concentration of 363 mM) (EL1), a fraction eluted with a solution with a mixing ratio of buffer C : buffer D of 1 : 1 (NaCl concentration of 575 mM) (EL2), a fraction eluted with a solution with a mixing ratio of buffer C : buffer D of 1 : 3 (NaCl concentration of 788 mM) (EL3), and finally, a fraction eluted with only buffer D (NaCl concentration of 1000 mM) (EL4) were each collected.

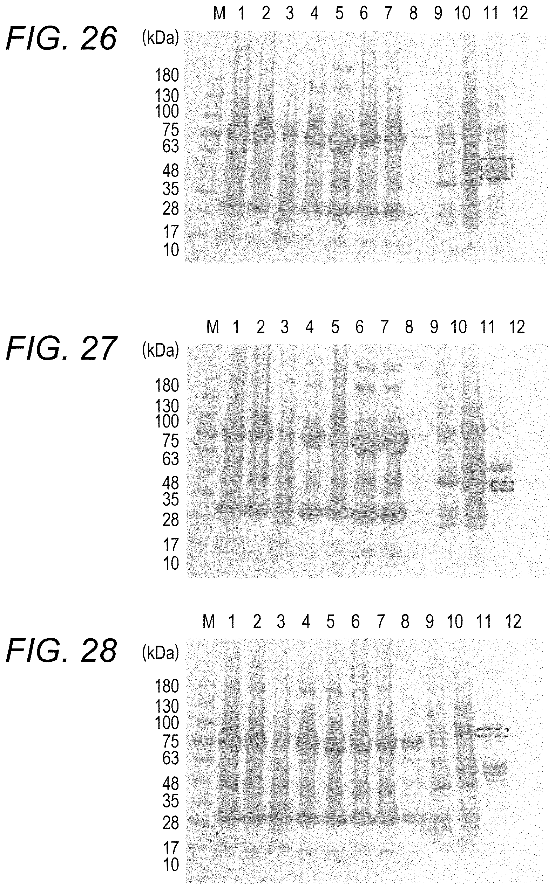

The same procedures were carried out as in Example 1 to detect DED-HRV3Csite-NQO1. The results were shown in Fig. 26. In Fig. 26, the results of electrophoresis are shown as follows; M: molecular weight marker, lane 1: homogenate solution, lane 2: supernatant fraction after centrifugation, lane 3: precipitated fraction after centrifugation, lanes 4 to 7: flow-through fractions 1 to 4 of the ion exchange resin, lane 8: elution fraction by buffer C (NaCl concentration of 150 mM) (EL0), lane 9: elution fraction by a 3 : 1 mixed solution of buffer C : buffer D (NaCl concentration of 363 mM) (EL1), lane 10: elution fraction by a 1 : 1 mixed solution of buffer C : buffer D (NaCl concentration of 575 mM) (EL2), lane 11: elution fraction by a 1 : 3 mixed solution of buffer C : buffer D (NaCl concentration of 788 mM) (EL3), and lane 12: elution fraction by buffer D (NaCl concentration of 1000 mM) (EL4). Explanation of each lane is common in Figs. 27 to 28.

### Example 25

The same procedures were carried out as in Example 7 to construct pHS01_DES_HRV3Csite_NQO1.

Furthermore, the same procedures were carried out as in Example 24 to express, purify and detect DES-HRV3Csite-NQO1. The results were shown in Fig. 27.

### Example 26

The same procedures were carried out as in Example 11 to construct pHS01_DED_HRV3Csite_Luciferase.

Furthermore, the same procedures were carried out as in Example 24 to express, purify and detect DED-HRV3Csite-Luciferase. The results were shown in Fig. 28.

### Example 27

The same procedures were carried out as in Example 7 to construct pHS01_DES_HRV3Csite_NQO1.

E. coli was transformed using pHS01_DES_HRV3Csite_NQO1, and 1.5 L of an LB medium containing ampicillin was cultured at 37°C and cooled to 15°C around a turbidity OD of about 0.6, then IPTG was added thereto to induce expression of protein. After 16 hours, bacteria were collected by centrifugation (8,000 g, 15 minutes) and frozen at -80°C. 120 mL of a buffer (20 mM PIPES (pH 6.1), 150 mM NaCl, 1 tablet Complete EDTA free (Roche)) was added to the frozen E. coli, and E. coli was crushed by ultrasonic wave. The crushed solution was centrifuged (15,000 g x 30 minutes), then the supernatant fraction was filtered with 0.8 uM of a filter agent, and the filtrate was collected. The filtrate was loaded on an ion exchange resin (Hitrap Q HP (1 mL)), and purified using a mixed solution of 20 mM PIPES (pH 6.1, 150 mM NaCl; buffer C) and 20 mM PIPES (pH 6.1, 1000 mM NaCl; buffer D). First, only buffer C (NaCl concentration of 150 mM) was flown through the ion exchange resin (EL0), and a fraction eluted with a 3 : 1 mixed solution of buffer C : buffer D (NaCl concentration of 363 mM) (EL1), a fraction eluted with a 1 : 1 mixed solution of buffer C : buffer D (NaCl concentration of 575 mM) (EL2), a fraction eluted with a 1 : 3 mixed solution of buffer C : buffer D (NaCl concentration of 788 mM) (EL3), and finally, a fraction eluted with only buffer D (NaCl concentration of 1000 mM) (EL4) were each collected.

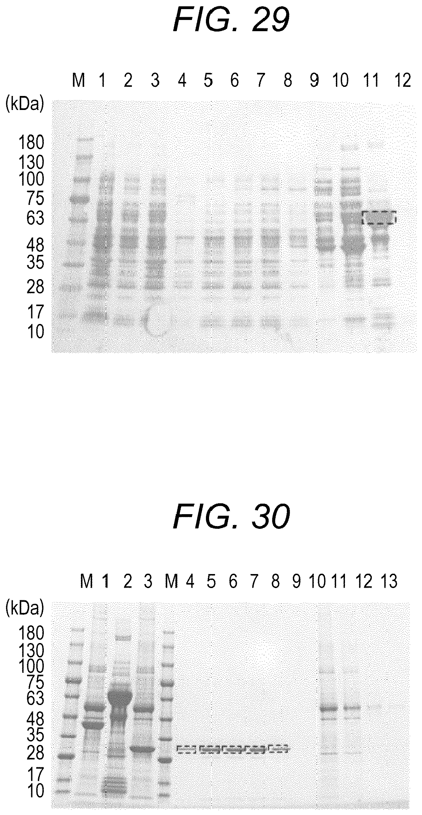

The same procedures were carried out as in Example 1 to detect DED-HRV3Csite-NQO1. The results were shown in Fig. 29. In Fig. 29, the results of electrophoresis are shown as follows; M: molecular weight marker, lane 1: homogenate solution, lane 2: supernatant fraction after centrifugation, lane 3: precipitated fraction after centrifugation, lanes 4 to 7: flow-through fractions 1 to 4 of the ion exchange resin, lane 8: elution fraction by buffer C (NaCl concentration of 150 mM) (EL0), lane 9: elution fraction by a 3 : 1 mixed solution of buffer C : buffer D (NaCl concentration of 363 mM) (EL1), lane 10: elution fraction by a 1 : 1 mixed solution of buffer C : buffer D (NaCl concentration of 575 mM) (EL2), lane 11: elution fraction by a 1 : 3 mixed solution of buffer C : buffer D (NaCl concentration of 788 mM) (EL3), and lane 12: elution fraction by buffer D (NaCl concentration of 1000 mM; EL4).

### Example 28

To EL3 of Example 24 was added a 1/10 volume of EL3 of Example 27, and the mixture was allowed to stand at 4°C. After 16 hours, 20 mM PIPES (pH 6.1) was added to the reaction solution to be diluted to a NaCl concentration of about 250 mM, and the diluted solution was loaded on an ion exchange resin (Hitrap Q HP (1 mL)) equilibrated with 20 mM PIPES (pH 6.1, 150 mM NaCl). Flow-through fractions were collected (Fr. 1 to 6 (NaCl concentration of about 250 mM)), and fractions finally eluted (Fr. 7 to 10) with only buffer D (NaCl concentration of about 1000 mM) were each collected.

The same procedures were carried out as in Example 1 to detect a target protein EQO1. The results were shown in Fig. 30. In Fig. 30, the results of electrophoresis are shown as follows; M: molecular weight marker, lane 1: EL3 of Example 24, lane 2: EL3 of Example 27, lane 3: a mixed reaction liquid of EL3 of Example 24, and EL3 of Example 27, lanes 4 to 8: flow-through fractions 1 to 5 of the ion exchange resin, and lanes 9 to 13: elution fractions 1 to 5 by buffer D.

### (Regarding Results of Examples 24 to 28)

Based on the results of Examples 24 to 28, it was shown that, in the expression and purification of the fusion protein and purification of the target protein, good separation is obtained even the pH of the buffer is lowered.

## Claims

1. A method for purifying a protein, comprising steps of:
preparing a sample comprising a fusion protein and contaminant proteins, the fusion protein comprising an amino acid sequence of a peptide tag and an amino acid sequence of a target protein, and
separating the contaminant proteins and the fusion protein,
wherein the peptide tag comprises 12 or more acidic amino acid residues.

2. The method for purifying a protein according to claim 1, wherein the peptide tag comprises any of the amino acid sequences of SEQ ID NOs. 1 to 8.

3. The method for purifying a protein according to claim 1 or 2, wherein the peptide tag comprises 18 or more acidic amino acid residues.

4. The method for purifying a protein according to any of claims 1 to 3, wherein the isoelectric point of the fusion protein is less than 6.

5. The method for purifying a protein according to any of claims 1 to 4, wherein the salt concentration of the sample is 50 mM or more and 500 mM or less.

6. The method for purifying a protein according to any of claims 1 to 5, wherein, in the sample preparation step,
a vector containing a polynucleotide encoding the fusion protein is introduced into a host cell, and the protein is expressed in the host cell to prepare the sample.

7. The method for purifying a protein according to any of claims 1 to 6, wherein the fusion protein further comprises a cleavable site recognized by a protease, between the amino acid sequence of a peptide tag and the amino acid sequence of the target protein.

8. The method for purifying a protein according to any of claims 1 to 7, wherein, in the step of separating the contaminant proteins, the fusion protein and the contaminant proteins are separated using an ion exchange resin.

9. The method for purifying a protein according to claim 8, wherein the ion exchange resin is an anion exchange resin.

10. The method for purifying a protein according to any of claims 1 to 9, wherein, the step of separating the contaminant proteins comprises
passing the sample through a anion exchange resin to obtain (i) the anion exchange resin to which the fusion protein is bound and (ii) a flow-through fraction comprising the contaminant proteins, and
eluting the fusion protein from the anion exchange resin using a buffer with a salt concentration of 600 mM or more.

11. The method for purifying a protein according to any of claims 1 to 10, after the step of separating the contaminant proteins,
further comprising a step of
separating the fusion protein into the target protein and the peptide tag in a solution, using the protease recognizing the cleavable site.

12. The method for purifying a protein according to claim 11, wherein, in the step of separating the fusion protein,
the solution comprising the separated target protein and the separated peptide tag is passed through an anion exchange resin, and
(i) the anion exchange resin to which the separated peptide tag is bound, and (ii) a flow-through fraction comprising the target protein are obtained.

13. The method for purifying a protein according to claim 10, wherein the salt concentration of the solution comprising the target protein and the peptide tag is 500 mM or less.

14. A fusion protein comprising an amino acid sequence of a peptide tag and an amino acid sequence of a target protein, the peptide tag comprising 12 or more acidic amino acid residues.

15. The fusion protein according to claim 14, wherein the peptide tag comprises any of the amino acid sequences of SEQ ID NOs. 1 to 8.

16. The fusion protein according to claim 14 or 15, wherein the peptide tag comprises 18 or more acidic amino acid residues.

17. The fusion protein according to any of claims 14 to 16, wherein the isoelectric point of the fusion protein is less than 6.

18. The fusion protein according to any of claims 14 to 17, wherein the fusion protein further comprises a cleavable site recognized by a protease, between the amino acid sequence of the peptide tag and the amino acid sequence of the target protein.

19. A method for producing the fusion protein according to any of claims 14 to 18, comprising steps of
introducing a polynucleotide encoding the amino acid sequence of the peptide tag and a polynucleotide encoding the amino acid sequence of the target protein into a host cell, and
expressing the fusion protein in the host cell.

20. Use of a peptide tag comprising 12 or more acidic amino acid residues, for the method according to any of claims 1 to 13.
